# EUROPEAN PATENT SPECIFICATION

(11) **EP 2 283 150 B1**
(45) Date of publication and mention of the grant of the patent: **02.01.2013**
(21) Application number: 09750087.0
(22) Date of filing: 21.05.2009
(51) Int. Cl.: C12Q 1/26

(54) **ASSAY FOR MONITORING ACTIVITY OF JMJD6**
TEST ZUR ÜBERWACHUNG DER AKTIVITÄT VON JMJD6
ESSAI POUR SUIVRE L'ACTIVITÉ DE JMJD6

(30) Priority: 21.05.2008 GB 0809262
(43) Date of publication of application: 16.02.2011
(73) Proprietor: ISIS Innovation Limited, Oxford, Oxfordshire OX2 7SG (GB); Ludwig-Maximilians-Universität München, 80539 München (DE)
(72) Inventor: SCHOFIELD, Christopher, Joseph, Oxford OX1 3TA (GB); WEBBY, Celia, Jane, Oxford OX1 3TA (GB); BOETTGER, Angelika, 82110 Germering (DE); WOLF, Alexander, 83026 Rosenheim (DE)
(74) Representative: Roques, Sarah Elizabeth
(86) International application number: PCT/GB2009/001270
(87) International publication number: WO 2009/141609

(56) References cited:
- WO-A2-2004/058252
- WO-A2-2006/034278
- WO-A2-2007/009044
- US-A1- 2004 146 964
- WEBBY C J ET AL: "Jmjd6 catalyses lysyl-hydroxylation of U2AF65, a protein associated with RNA splicing" SCIENCE 20090703 AMERICAN ASSOCIATION FOR THE ADVANCEMENT OF SCIENCE USA, vol. 325, no. 5936, 3 July 2009 (2009-07-03), pages 90-93, XP002538799
- WOLF ALEXANDER ET AL: "Changing story of the receptor for phosphatidylserine-dependent clearance of apoptotic cells" EMBO REPORTS, vol. 8, no. 5, May 2007 (2007-05), pages 465-469, XP002538800 ISSN: 1469-221X
- CIKALA MIHAI ET AL: "The phosphatidylserine receptor from Hydra is a nuclear protein with potential Fe(II) dependent oxygenase activity" BMC CELL BIOLOGY, BIOMED CENTRAL, LONDON, GB, vol. 5, no. 1, 11 June 2004 (2004-06-11), page 26, XP021001213 ISSN: 1471-2121

## Description

### Field of the Invention

The present invention relates to assays for monitoring activity of Jmjd6 activity, in particular, to assays for identifying modulators of Jmjd6 activity. The present invention also relates to the treatment of disorders associated with abnormal RNA splicing, such genetic disorders and cancer.

### Background to the Invention

Metazoan cells respond to limiting oxygen by activation of the hypoxia inducible factor (HIF) system. The activity and lifetime of the HIFα subunit are regulated by oxygen dependent post-translational hydroxylation; prolyl-hydroxylation signals for HIFα degradation via the ubiquitin-proteasome machinery and asparaginyl-hydroxylation reduces the HIF transcriptional activity by blocking its interaction with p300. HIF prolyl and asparaginyl (FIH, factor inhibiting HIFα) hydroxylases are Fe(II) and 2-oxoglutarate (20G) oxygenases. The role of the HIF hydroxylases in transcriptional regulation has raised the question of whether there are other direct interfaces between oxygen levels and the regulation of gene expression.

### Summary of the Invention

The present inventors have found that Jmjd6 (also known as the phosphatidylserine receptor) is a 2-oxoglutarate (20G) oxygenase catalysing lysyl-hydroxylation of mRNA splicing regulatory proteins involved in protein synthesis. The present inventors have also demonstrated the involvement of Jmjd6 in the regulation of mRNA splicing. Modulation of the activity of Jmjd6 can, therefore, be used to regulate mRNA splicing (and therefore protein biosynthesis). Inhibitors of Jmjd6 lysyl hydroxylase activity have also been identified by the inventors.

Accordingly, the present invention provides a method for assaying Jmjd6 activity, the method comprising contacting a splicing regulatory protein, or a fragment or variant thereof comprising an RS domain and a lysine residue, with a Jmjd6 polypeptide and determining whether the splicing regulatory protein, or fragment or variant thereof, is hydroxylated.

The splicing regulatory protein or fragment thereof and Jmjd6 polypeptide are typically contacted in the presence of Fe(II) and 2-oxoglutarate and optionally in the presence of a reducing agent or other factors that optimise catalytic activity. The splicing regulatory protein may, for example, be the splicing factor U2AF 65 kDa subunit (U2AF65), Luc7-like2 or cisplatin resistance-associated overexpressed protein (CROP), or a fragment of any thereof comprising a lysine residue. The fragment of the splicing regulatory protein is typically rich in arginine and serine residues and comprises at least one lysine residue. The Jmjd6 polypeptide comprises the amino acid sequence of SEQ ID NO: 1 or is a fragment or variant thereof having lysyl hydroxylase activity.

The assay may be carried out in the presence of a test agent to determine whether the test agent is a modulator of Jmjd6 activity. The method may further comprise determining whether the test agent modulates activity of a 2-oxoglutarate dependent oxygenase other than Jmjd6, thereby determining whether the test agent selectively modulates Jmjd6 activity or selectively modulates activity of the 2-oxoglutarate dependent oxygenase other than Jmjd6.

The invention also provides a method for identifying a modulator of RNA splicing, the method comprising contacting a cell which expresses Jmjd6 with a test agent and determining whether the test agent modulates Jmjd6 regulation of RNA splicing. In this method the cell may comprise a RNA splicing reporter construct and the method comprises determining whether Jmjd6-mediated regulation of RNA splicing of the reporter construct is modulated by the test agent.

The test agent tested in any method of the invention may be a reported inhibitor of a 2-OG oxygenase other than Jmjd6, or an analogue or variant of such an inhibitor. For example, the inhibitor may be an N-oxalyl amino acid such as N-oxalylglycine or a derivative thereof, a glycine or alanine derivative, a 2-oxoacid analogue, a flavonoid or flavonoid derivative such as genistein.

### Brief Description of the Figures

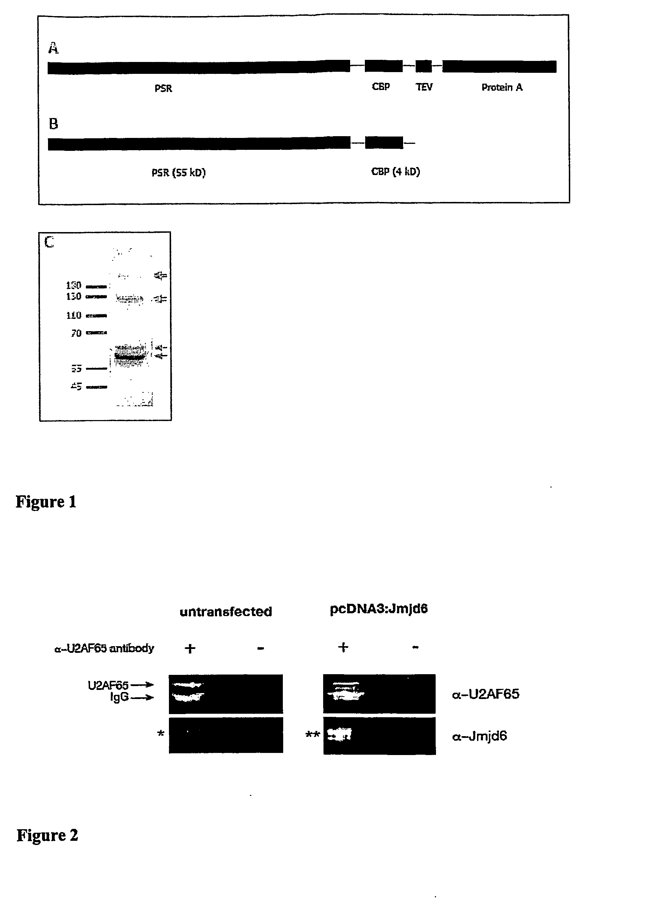

Figure 1 illustrates the tandem affinity purification of Jmjd6 from HEK293T-cells. (A) Schematic representation of TAP-tagged Jmjd6 fusion protein: the tag represents a fusion of calmodulin binding peptide (CBP), a TEV cleavage site and Protein A. (B) Schematic of remaining Jmjd6-CBP fusion protein after two purification steps including affinity chromatography on an immunoglobulin column, removal from the column with TEV and affinity chromatography on a calmodulin column. (C) Coomassie stained SDS-PAGE gel of tagged Jmjd6-protein purified from HEK293 cells. Arrows indicate bands that were identified as Jmjd6 by MALDI-TOF.
Figure 2 shows the results of a U2A65 immunoprecipitation experiment in HeLa cell lysates expressing endogenous Jmjd6 (left panel) or over-expressing Jmjd6 from pcDNA3/Jmjd6 plasmid (right panel). Samples were analysed on Western blot probed with anti-U2AF65 antibody (top panel) and anti-Jmjd6 antibody (lower panel). -/+ refers to the absence and presence of anti-U2AF65 antibody; * is endogenous Jmjd6; ** is over-expressed Jmjd6; IgG is the heavy chain of immunoglobulin.

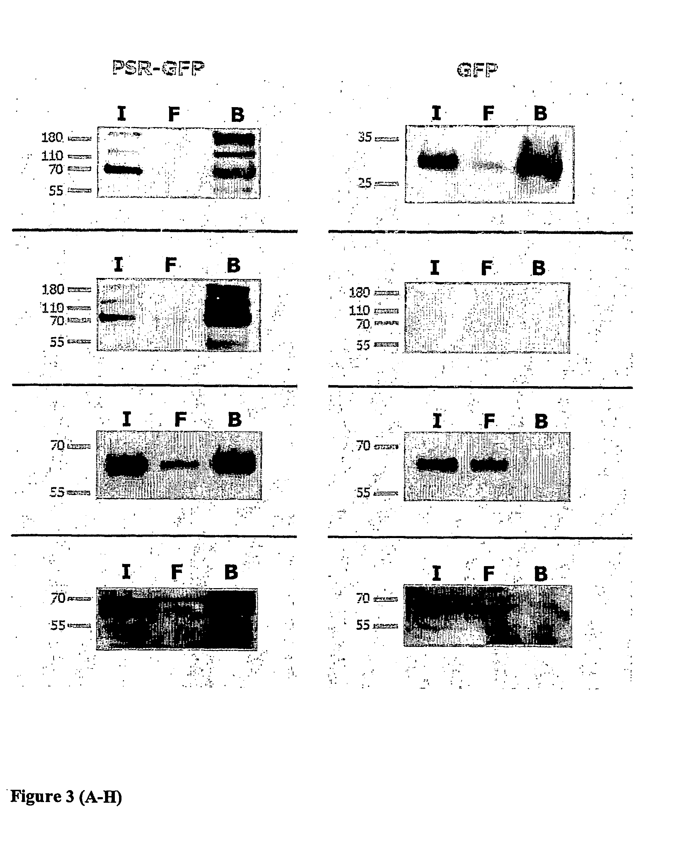

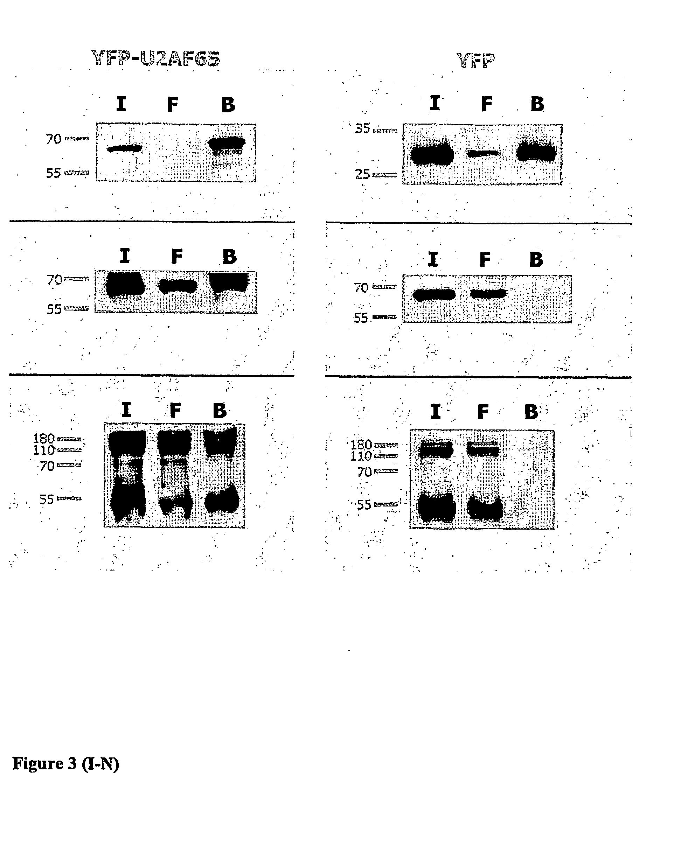

Figure 3 shows the results of immunoprecipitation analysis of the interaction of Jmjd6 with U2AF65 and CROP in HEK293 cells. Lysates from HEK293 cells expressing Jmjd6-GFP (left hand panels) or GFP (right hand panels) were subjected to GFP-pulldown experiments. Input (I), flowthrough (F) and beads (B) fractions were analysed by SDS-PAGE/Western blotting with anti-GFP antibody (A, B), anti-Jmjd6 antibody (C, D), anti-U2AF65 antibody (E, F) or anti-CROP antibody (G, H). Lysates from HEK293 cells overexpressing untagged Jmjd6 and YFP-U2AF65 (left hand panels) or YFP (right hand panels) were subjected to YFP-pulldown experiments. Input (I), flow through (F) and beads (B) fractions were probed with anti-GFP antibody (I, J), anti-U2AF65 antibody (K, L) and anti- Jmjd6 antibody (M, N). Occurence of a band in the beads fraction indicates precipitation by the binder or co-precipitation with the GFP/YFP tagged proteins.

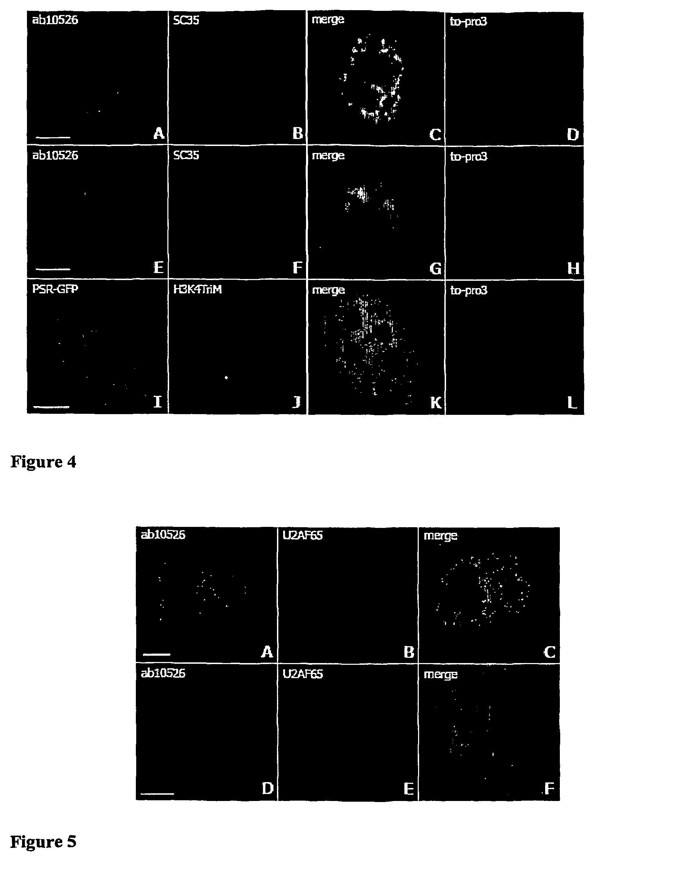

Figure 4 shows the results of localisation experiments of endogenous Jmjd6. Jmjd6 is localised in nuclear speckles: it is partially co-localised with SC-35, but is not colocalised with euchromatin. A-H: Immunofluorescence of HeLa cells co-stained with anti-Jmjd6 antibody ab10526 (Abcam) and anti-SC-35 antibody (A-D, enlargement E-H). I-L: Hela cells transfected with Jmjd6-GFP and co-stained with anti-H3K9 (trimethylated). Confocal sections, merged images (C,G,K); counterstaining with DNA dye To-Pro3 (D,H,L), A,I: scale bar 5 µm E: scale bar 2µm.
Figure 5 shows the results of localisation experiments of Jmjd6 and U2AF65. Jmjd6 is partially co-localised with U2AF65. Immunofluorescence of HeLa cells co-stained with anti-Jmjd6 antibody ab10526 (A, D) and anti-U2AF65 antibody (B, E). Confocal sections, merged images (C,F), A-C: scale bar 5 µm, D-E, enlargements, scale bar 2 µm.

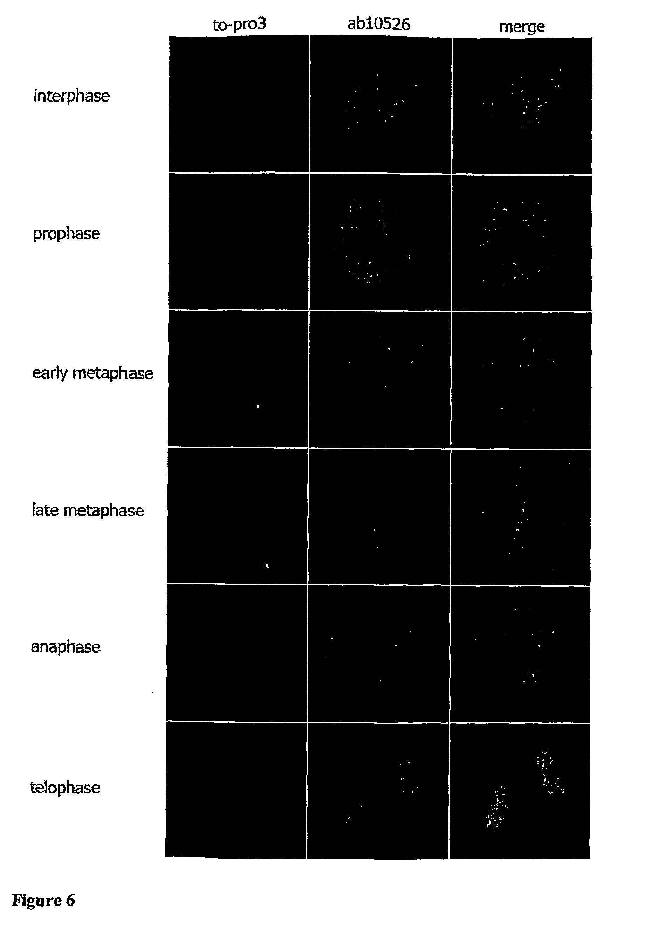

Figure 6 shows the localisation of Jmjd6 in all phases of the cell cycle. Jmjd6 is redistributed during the cell cycle. Immunofluorescence of HeLa cells in indicated phases of the cell cycle is stained with anti-Jmjd6 antibody ab10526 (middle panel) and counterstained with DNA dye To-Pro3 (left hand panel), merged images in right hand panel.

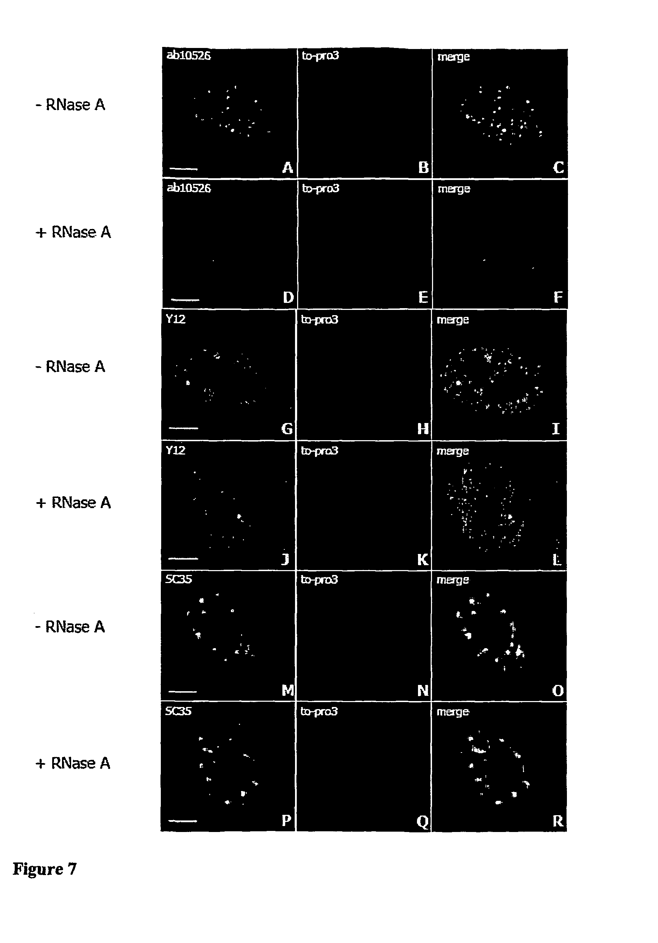

Figure 7 shows the localisation of Jmjd6 in the presence of RNAse. Jmjd6 localisation changes after RNAse treatment. Immunofluorescence of Hela cells stained with anti-Jmjd6 antibody ab10526 (E-F), anti-Sm antibody Y12 (G-L) and anti-SC-35 antibody SC-35 (M-R), (left hand panel). Cells were treated with RNAseA in PBS (+RNAse A) or PBS (-RNAse A). Counterstaining with TO-PRO3 (middle panels), merged images (right hand panel), confocal sections, scale bar 5µm.

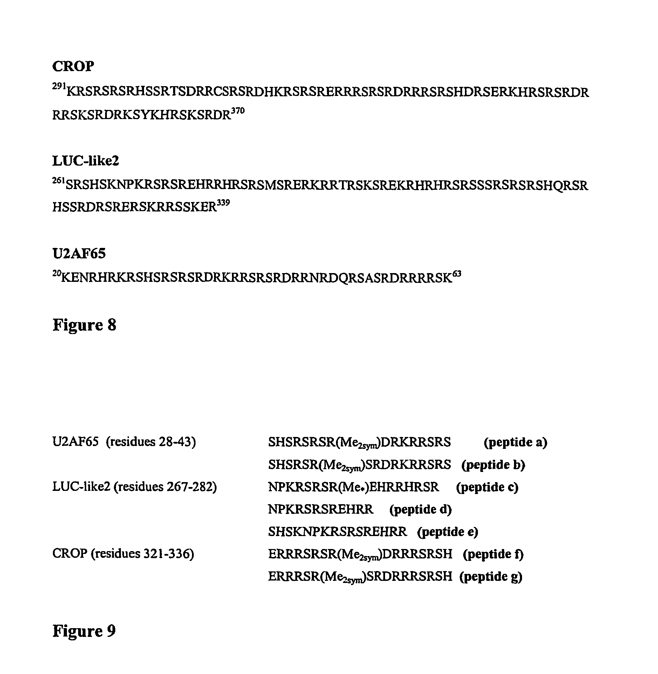

Figure 8 shows the amino acid sequences of the RS domains of CROP, Luc-like2 and U2AF65.
Figure 9 shows peptides from U2AF65, CROP and LUC-like2 RS domains; sym represents symmetrical dimethylated arginine and * indicates symmetrical, asymmetrical and mono methylated arginines were incorporated at this position in the peptide.

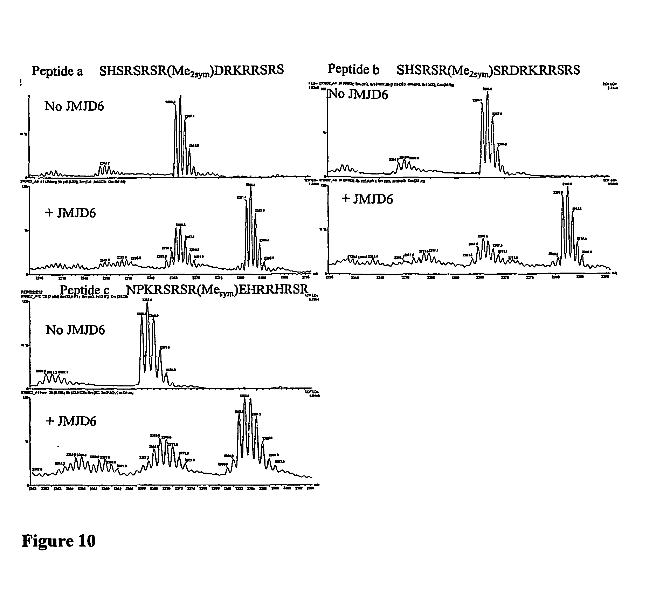

Figure 10 shows the Matrix Assisted Laser Desorption Ionisation time-of-flight (MALDI TOF) analysis of peptides a, b and c in the presence of Fe(II), 2OG, ascorbate and Jmjd6. The additional +222 mass to each peptide is due to the addition of a Fmoc protecting group at the N-terminus of each peptide, this was carried through from the synthesis.

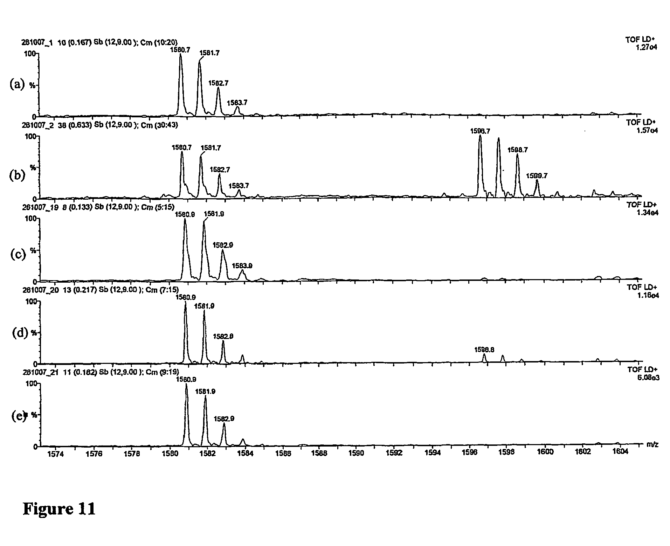

Figure 11 shows the MALDI TOF analysis of Fe(II)- and 20G- dependent activity of Jmjd6. (a) Peptide d (100 µM) incubated with ascorbate (100 µM) and Fe(II) (100 µM) (no JMJD6). (b)-(d) Incubation of JMJD6 (10 µM) with peptide d in the presence of ascorbate and (b) 2OG, Fe(II), (c) Fe(II) (no 2OG) and (d) 2OG (no Fe(II)). (e) Incubation of JMJD6 with peptide d in ascorbate, 2OG, Fe(II) and NOG (120 mM). Note in (d) the enzyme (400 µM) was pre-treated with EDTA (34 mM) overnight and then desalted before addition to the assay.

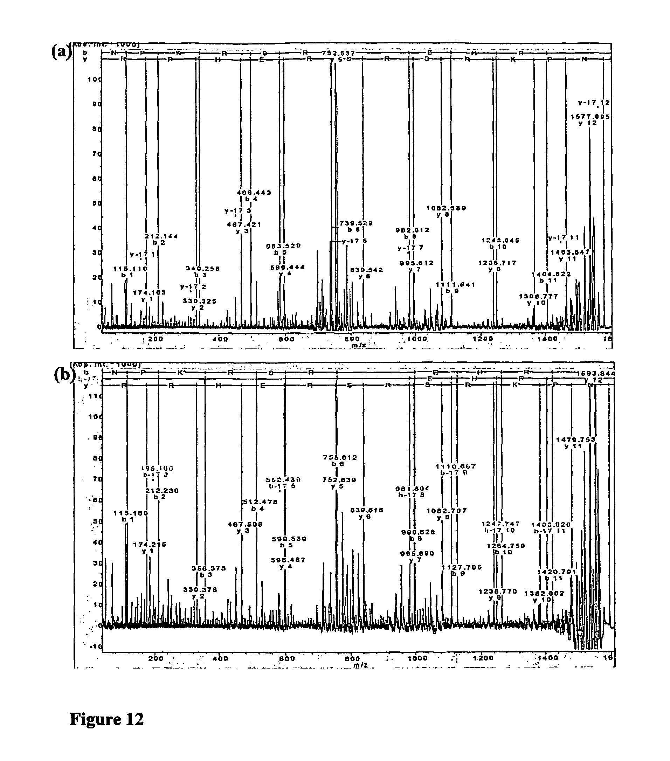

Figure 12 shows the MS/MS spectra from post source decay (PSD) fragmentation of unmodified (a) and hydroxylated (b) peptide d. The 'b' and 'y' series of fragmented ions are labelled with the mass and the corresponding residue.

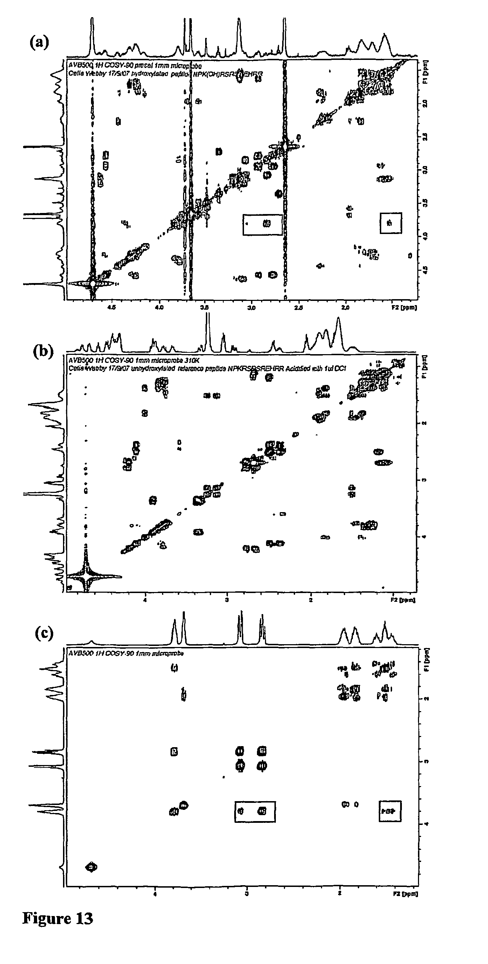

Figure 13 shows the results of a 2D COSY experiment comparing hydroxylated peptide d (a) with unhydroxylated peptide d (b) and DL and DL allo-hydroxylysine (c). The new resonances in the hydroxylated peptide d are shown in a box and are consistent with resonances observed in the hydroxylysine reference experiment.

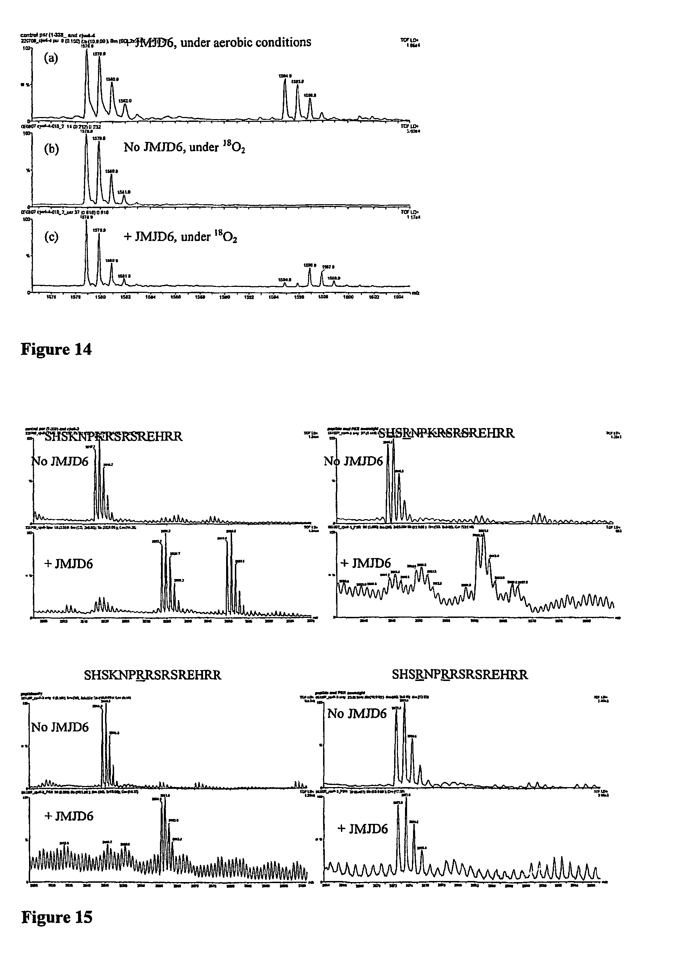

Figure 14 shows the MALDI TOF mass spectrum of peptide d after incubation with Jmjd6 under ¹⁸O₂. (a) Incubation of peptide d (100 µM) under aerobic conditions with JMJD6 (10 µM), (b) incubation of peptide d without (b) and with (c) JMJD6 under ¹⁸O₂ conditions.
Figure 15 shows the MALDI TOF analysis of peptide e and variations of peptide e with Jmjd6. Incubation of Jmjd6 (10 µM) with peptides (100 µM) e (a), e2 (b), e3 (c) and e4 (d) in the presence of ascorbate (100 µM), 2OG (500 µM) and Fe(II) (100 µM).

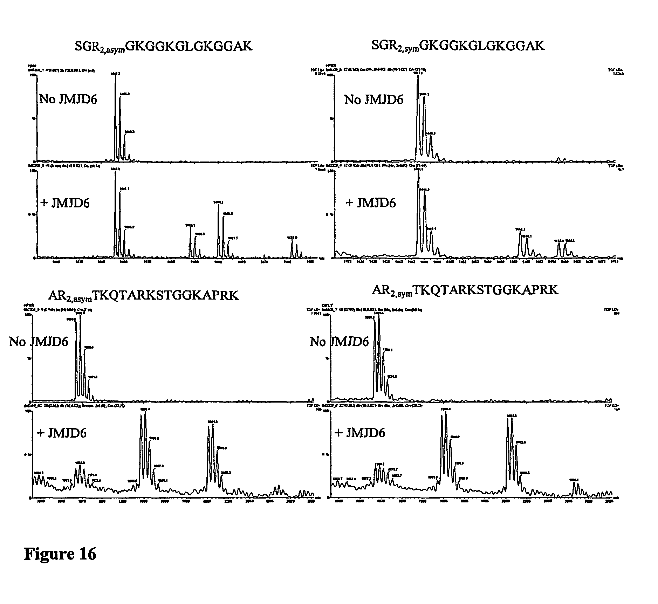

Figure 16 shows the MALDI TOF analysis of histone peptides with Jmjd6. Incubation of JMJD6 (10 µL) with H3R2 peptides (AR*TKQTARKSTGGKAPRK) and H4R3 peptides (SGR*GKGGKGLGKGGAK) (100 µM) (where * is both asymmetrically dimethylated and symmetrically dimethylated) in the presence of ascorbate (100 µM), 2OG (500 µM) and Fe(II) (100 µM).

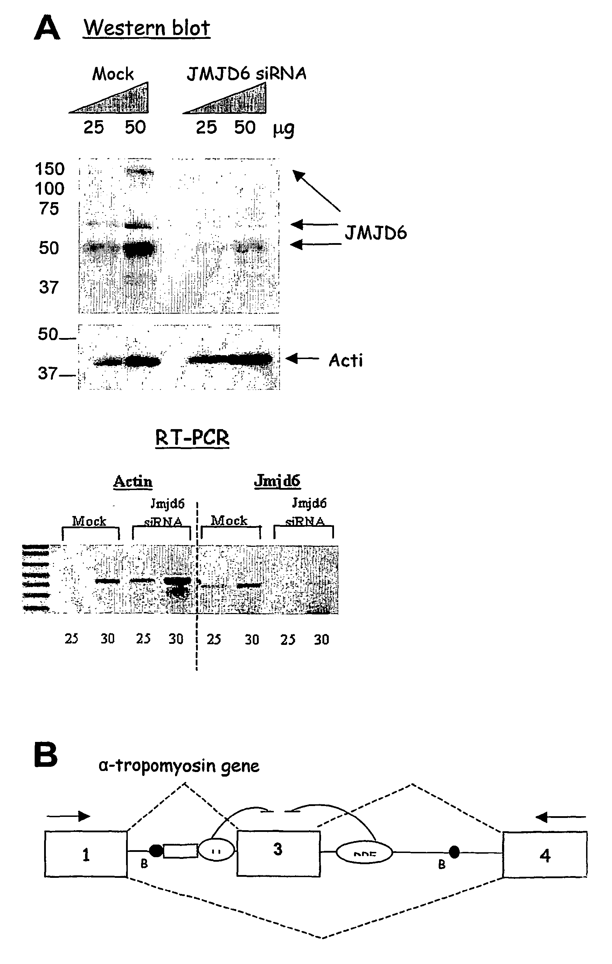

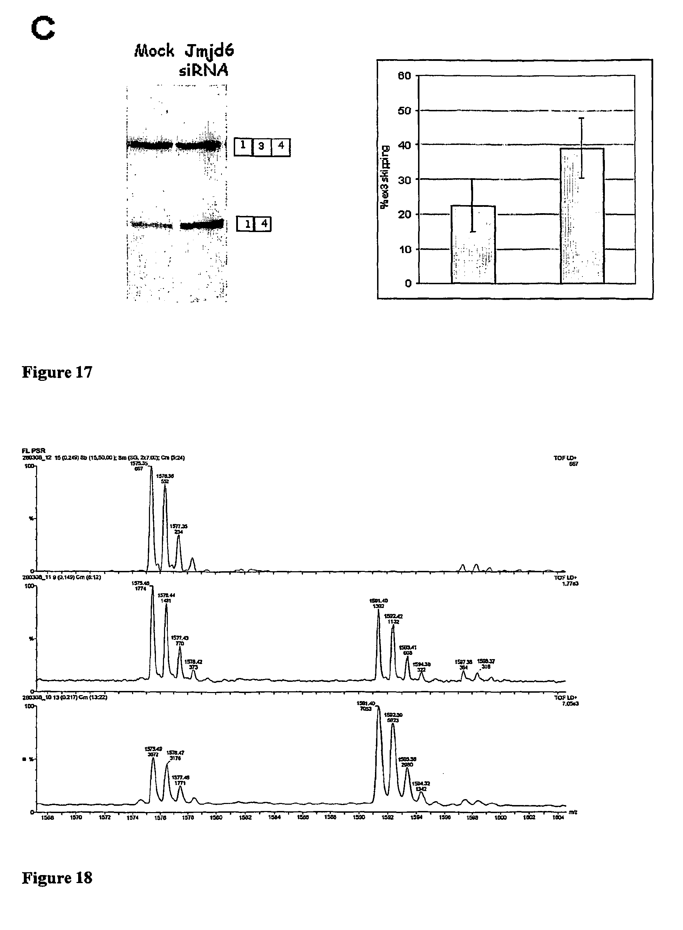

Figure 17 shows that RNAi-mediated depletion of Jmjd6 affects α-tropomyosin alternative splicing. (A) RNAi-mediated depletion of human JMJD6 in HeLa cells. Left-hand panel shows the Western blot analysis with anti-JMJD6 and anti-actin antibody carried out on 25 and 50 µg of total protein extract from Mock and JMJD6 siRNA-treated HeLa cells. Right-hand panel shows Real Time-Polymerase Chain Reaction (RT-PCR) analysis of β-actin and Jmjd6 mRNAs in mock and Jmjd6 siRNA-treated cells. Numbers below the gel represent the PCR cycles. (B) Diagram of α-TM minigene construct, with exons 1, 3 and 4 shown as boxes and introns as lines. Branch point (BP) sequences of exon 3 and 4 are black ovals. Splicing patterns are represented as diagonal dashed lines. In most cells the generated splicing product is 1-3-4, while in smooth muscle (SM) cells it is 1-4. Polypyrimidine tract (PPT), Downstream and Upstream Regulatory Elements (DRE and URE) are involved in the repression of exon 3 in SM cells. α-TM minigene construct contains 6 tandem URE copies to increase skipping of exon 3 in HeLa cells (1). Arrows indicate the location of primers used in RT-PCR analysis. (C) RT-PCR analysis of α-TM minigene construct transfected in mock-treated and PSR-depleted HeLa cells. The splicing products 1-3-4 and 1-4 were resolved on 6% polyacrylamide gel. Histogram shows the average percentage of α-TM exon 3 skipping (+/- SD) based on three independent experiments.
Figure 18 shows the results of a standard hydroxylation assay with peptide d (100 µM) (a) in the absence of Jmjd6; (b) in the presence of C-terminally truncated (residues 1-343) Jmjd6 (160 µM); and (c) in the presence of full-length (160 µM) Jmjd6.

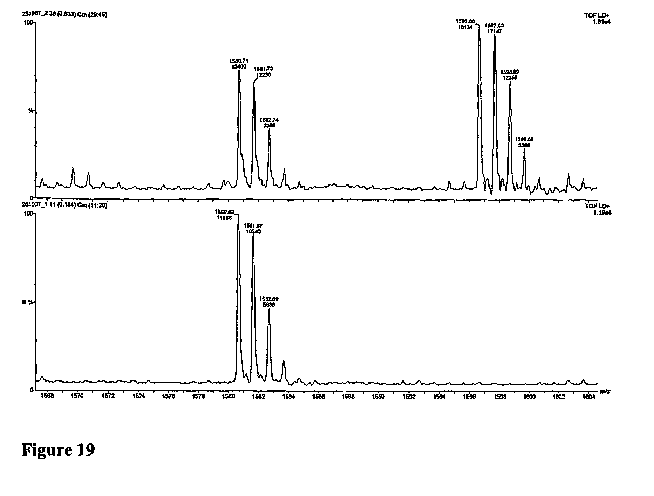

Figure 19 shows hydroxylation of peptide d by C- and N- terminally truncated Jmjd6 (residues 25-338)(a). (b) is a negative control excluding Jmjd6.

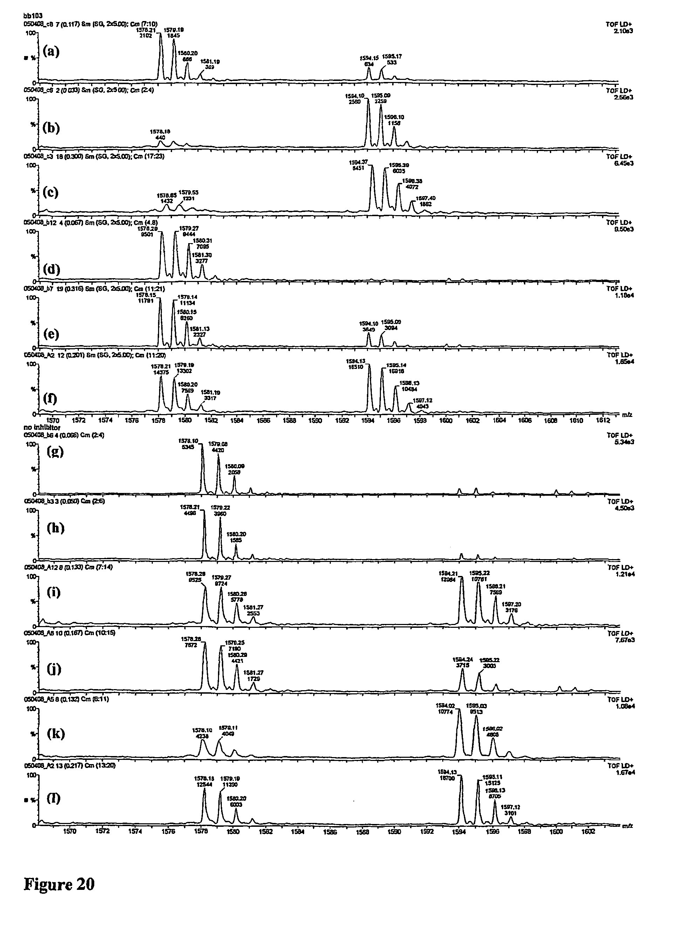

Figure 20 shows inhibition of Jmj6 hydroxylation of peptide d. (f) and (1) are control assays without inhibitors; (a) with the addition of FG0041; (b) 2,6-PDCA; (c) 2,5-PDCA; (d) 2,4-PDCA; (e) LBE-6-3; (g) succinate; (h) fumarate; (i) NOFD; (j) FG2216; (k) NOG.

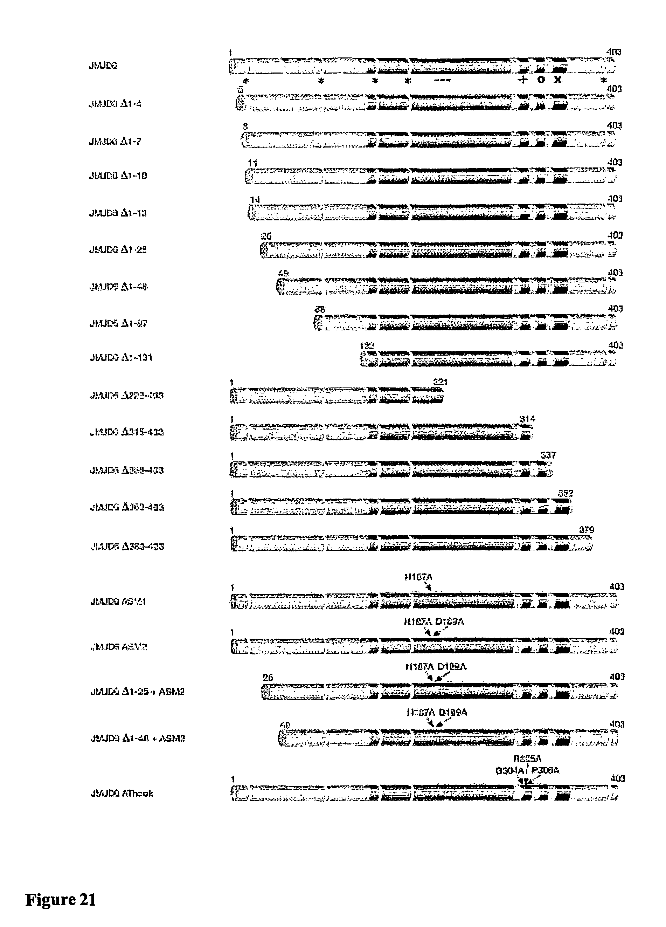

Figure 21 shows a diagram of Jmjd6 mutants that were used in several of the Examples. Light rings (*): NLS; medium grey (---) Jmj-domain; dark grey(+):AT-hook; dark grey(o): sumoylation site; dark grey (x): poly-S-region.

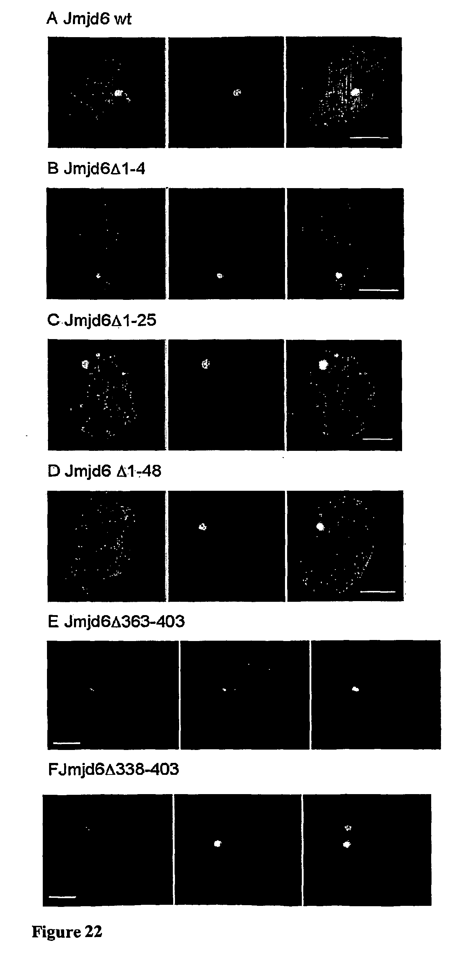

Figure 22 shows fluorescence two hybrid assay with pmCherry-Jmjd6-Lac-inhibitor dots (middle panel in A-D, left hand panel in E, F) and Jmjd6-GFP fusion proteins with deletions in Jmjd6 as indicated above each panel (left hand panels in A-D, middle panel in E, F). Right hand panels constitute merged images, in A-D including the DNA-label with DAPI. Shown are confocal images from single optical sections, scale bars 5 µm.

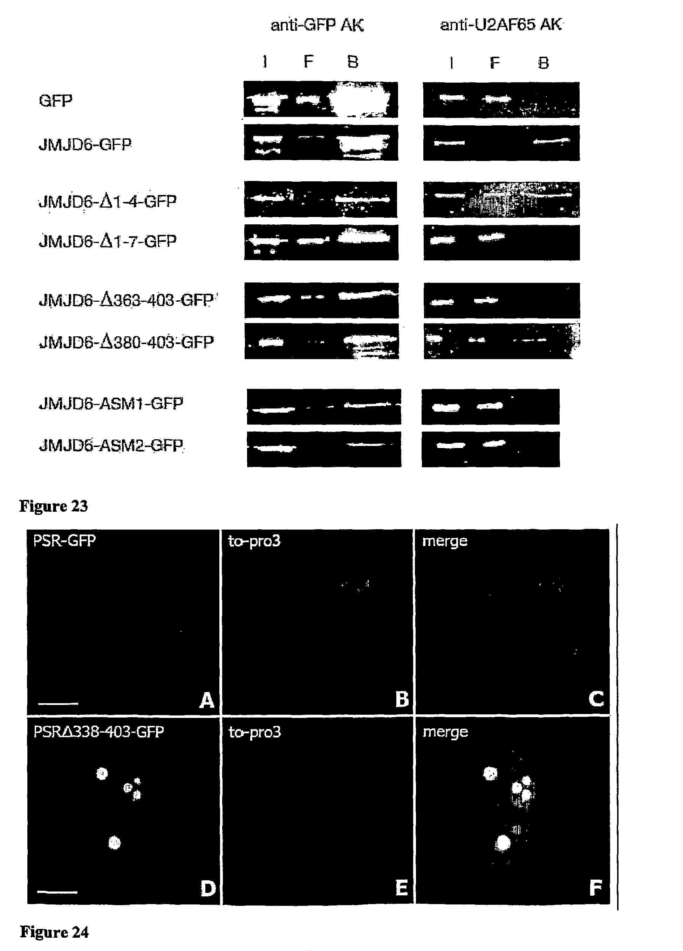

Figure 23 shows western blots of precipitates after GFP pulldown from lysates containing the indicated GFP-Jmjd6 variants. Left hand panels: blots stained with anti-GFP antibody to show successful pulldown of the fusion proteins; right hand panels: blots are stained with anti U2AF-65 antibody to show U2AF-65 that was co-precipitated due to its interaction with Jmjd6. I = input, proteins in the lysate, F = flowthrough, proteins not bound to the GFP binder, B = bound, proteins precipitated with the GFP binder and captured with protein A beads.
Figure 24 shows Jmjd6-GFP (A-C) and the C-terminal deletion mutant Jmjd6Δ338-403 (D-F) expressed in HeLa cells (left hand channel) and counterstained with DNA dye to-pro3 (middle panel). Right hand panel shows merged images. Single sections of confocal laser microscopic images, scale bar 5µm.

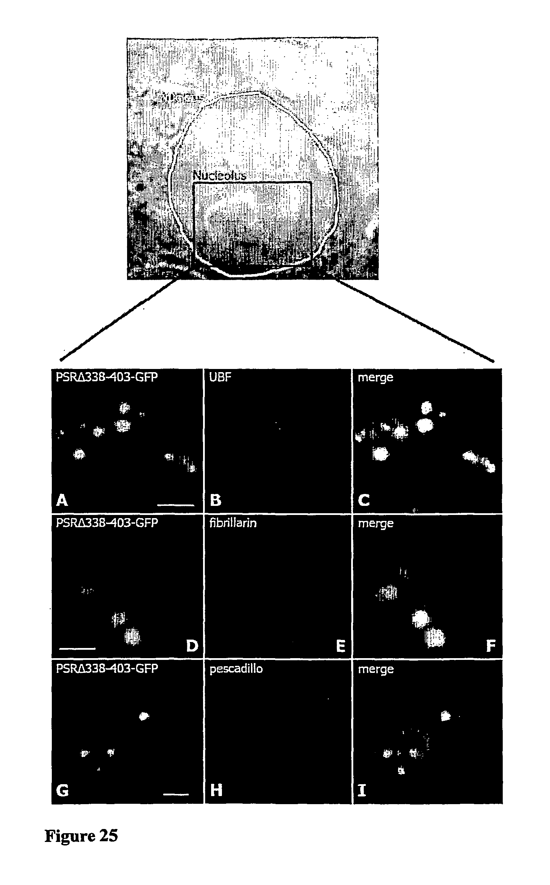

Figure 25 shows the nucleoli of cells overexpressing GFP- Jmjd6Δ338-403 (A, D, G), co-stained with anti-UBF antibody (B), anti-fibrillarin antibody (E) and anti-pescadillo antibody (H). Right-hand panel shows merged images, confocal sections, scale bar 5µm.

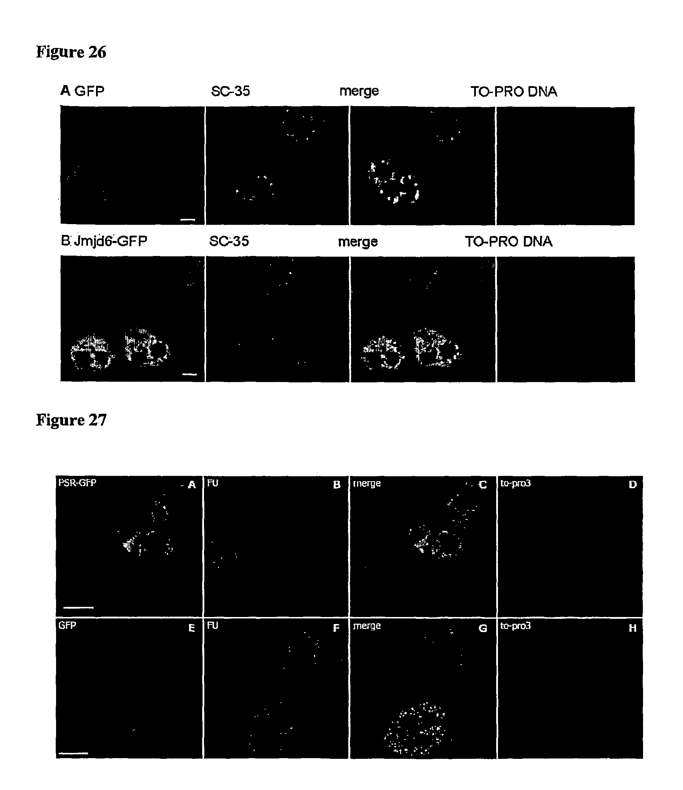

Figure 26 shows HeLa cells overexpressing GFP(A) or Jmjd6-GFP(B) co-stained with anti SC-35 antibody (as indicated above the panels). Merged images and DNA counterstaining are also shown. Scale bar 5µm. Single sections of confocal laser microscopic images are shown.
Figure 27 shows HeLa cells after transfection with plasmids encoding Jmjd6-GFP (A-C) or GFP (E-H) and labelled with 5 FU (B, F). They were also counterstained with the DNA-dye to-pro3 (D, H). C and G show merged images from the GFP and the 5 FU signals. Optical sections of confocal microscopic images are shown, scale bar 5µm.

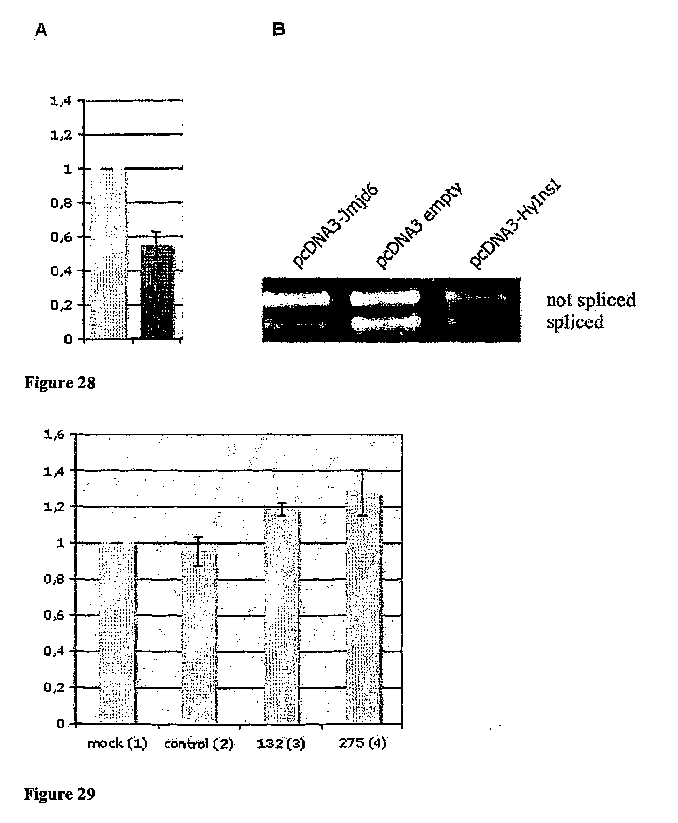

Figure 28 shows double reporter splice assays. A: relative activities of luciferase to β-galactosidase activities in mock transfected (left bar) and Jmjd6 overexpressing HeLa cells (right bar). Luciferase activity decreases in the absence of splicing due to a stop-codon wihin the intron. B: RT-PCR specifically amplifying spliced and non-spliced reporter cDNA from cells transfected with indicated plamids.
Figure 29 shows double reporter splice assay; relative activities of luciferase to β-galactosidase activities in cells after transfection without (mock) or with inidicated siRNAs.

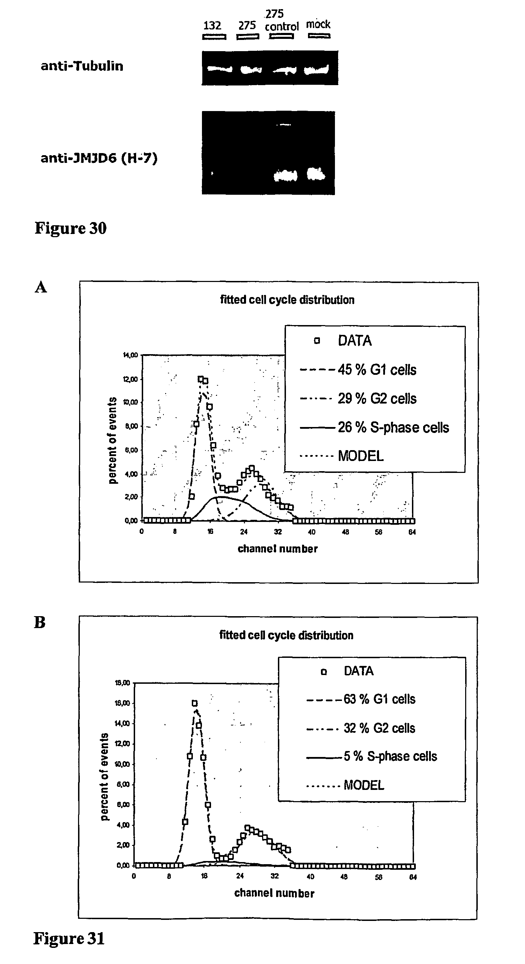

Figure 30 shows western blots after SDS-PAGE from HeLa cell lysates transfected with Jmjd6 specific siRNAs (132 and 275) and not Jmjd6 related siRNA (275 control). Upper panel: tubulin staining indicating equal loading of lysates onto lanes; lower panel: anti Jmjd6 staining (antibody H7), loss of Jmjd6 after treatment with Jmjd6 specific RNAis in lanes 1 and 2.
Figure 31 shows FACS-cell cycle analysis of HeLa cells after transfection with plasmid for expression of Jmjd6-PSR. Data were aquired with Partec Flowmax. DAPI fluorescence (DNA content) of GFP positive and negative cells was analysed separately. Profile for GFP negative, non-GFP-Jmjd6 overexpressing cells is shown in A; profile from GFP positive and thus GFP-Jmjs6 overexpressing cells are shown in B.

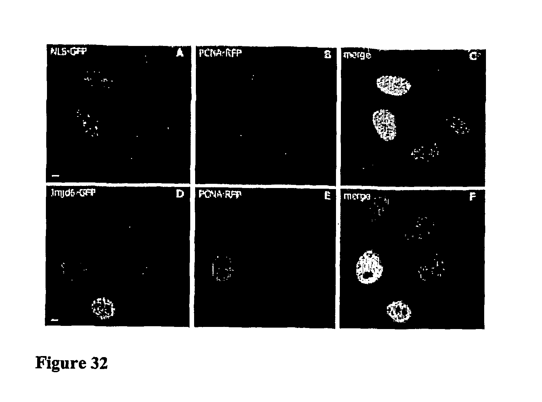

Figure 32: A-C show co-expression of PCNA-RFP and NLS-GFPJmjd6-GFP in HeLa cells. Punctate PCNA pattern indicates S-phase. D-F show co-expression of PCNA-RFP and Jmjd6-GFP: cells do not show PCNA S-phase pattern.

### Brief Description of the Sequences of the Informal Sequence Listing

SEQ ID NO: 1 is the amino acid sequence of full-length Jmjd6.
SEQ ID NO: 2 is the amino acid sequence of a C-terminally truncated Jmjd6 polypeptide (residues 1 to 343 of SEQ ID NO: 1).
SEQ ID NO: 3 is the amino acid sequence of a C- and N-terminally truncated Jmjd6 polypeptide (residues 25 to 338 of SEQ ID NO: 1).
SEQ ID NO: 4 is the amino acid sequence of U2AF65.
SEQ ID NO: 5 is the amino acid sequence of Luc7-like2.
SEQ ID NO: 6 is the amino acid sequence of CROP.

### Detailed Description of the Invention

The present inventors have shown that Jmjd6 interacts with several proteins involved in protein expression, in particular with proteins involved in RNA metabolism, processing and splicing. The present inventors have also shown that Jmjd6 is mainly localised in the nucleoplasm where it is partially co-localised with the non-snRNA spliceosome component SC-35 and is partially co-localised with the splicing factor U2AF 65 KDa subunit (U2AF65). The present inventors have also shown that interaction of Jmjd6 with U2AF-65 depends on Jmjd6 N-terminal and C-terminal sequences.

The present inventors have demonstrated that Jmjd6 is also partially localised in the nucleolus, where it co-localises with the nucleolar transcription factor, UBF (Upstream Ribosomal Binding Factor). Nucleolar localisation (specifically in the fibrillar centre) is increased using a C-terminal deletion of Jmjd6 (Jmjd6Δ338-403).

The present inventors have demonstrated that Jmjd6 hydroxylates lysine residues in the splicing regulatory proteins and other proteins involved in protein expression. In particular, the inventors have shown that lysine residues present in or near the RS domains of these peptides are hydroxylated by Jmjd6. Lysine hydroxlation has been shown by the inventors to occur at the C5 position with R stereochemistry.

The present inventors have also demonstrated a link between Jmjd6 and RNA splicing in cells in culture and *in vivo.* Jmjd6 has been shown, by the present inventors, to be involved in regulating constitutive and alternative splicing. The inventors have shown that over-expression of Jmjd6 in cells leads to an inhibition of mRNA splicing and that reducing expression of Jmjd6 increases exon skipping during alternative splicing and increases constitutive splicing.

The present inventors have also shown that Jmjd6 overexpression causes diassembly of SC-35 speckles, suggesting a connection between Jmjd6 activity and phosphorylation of splice factors, and global inhibition of 5-FU labelled native transcripts.

The present invention provides a method for assaying Jmjd6 activity, the method comprising contacting a protein involved in protein synthesis, preferably a protein involved in RNA metabolism, processing or splicing, or a fragment or variant thereof comprising a lysine residue, with a Jmjd6 polypeptide and determining whether the protein or fragment thereof is hydroxylated.

The protein involved in protein synthesis, preferably a protein involved in RNA metabolism, processing or splicing, more preferably a splicing regulatory protein, is one that interacts with Jmjd6 *in vitro* and/or *in vivo.* The protein is typically rich in Arg (R) and Ser (S) residues and preferably comprises a RS domain. The RS domain may comprise an imperfect 8-amino acid repeat motif, typically having the consensus sequence SRSRXRRR, wherein X is Asp (D) or Glu (E). The RS domain may also comprise one or more Lys (K), His (H), Asn (N) and/or Pro (P) residue, preferably at least one K residue. Suitable proteins for use in an assay of the invention may be identified by determining whether the protein interacts with full-length Jmj d6 and/or by determining whether the protein, or a fragment thereof comprising at least one K residue, is hydroxylated by Jmjd6.

Examples of suitable proteins for use as Jmjd6 substrates in a method of the invention include U2AF 65 KDa subunit (U2AF65), Luc7-like2, cisplatin resistance-associated over-expressed protein (CROP), ATP-dependent RNA helicase DDX41, Poly-U binding splicing factor, Luc7-like-1, Splicing factor arginine/serine rich 11 (p54), Arginine/serine rich coiled-coil protein 1, ATP-dependent RNA helicase DDX46, RNS binding motif protein 25, Acinus (Apoptotic chromatin condensation inducer in the nucleus), ATP-dependent RNA helicase DDX17 (p72), RNA binding protein, Pre-mRNA-processing factor 40 homolog A, Splicing factor U2AF 35 KDa subunit (U2AF35), RNA-binding protein 39 (Splicing factor HCC1), RNA polymerase-associated protein RTF1 homolog, Cleavage and polyadenylation specificity factor 6, Nucleolar RNS helicase II (DDX21), Nuclear phosphoprotein p130, Treacle protein (Treacher Collins syndrome protein), H/ACA ribonucleoprotein complex subunit 4 (Dyskerin), Nuclease sensitive element binding protein 1. DNA-binding protein A, Parahbromin, Bromodomain-containing protein 4, Eukaryotic translation initiation factor 3 subunit 4, Eukaryotic translation initiation factor 3 subunit 8, Elongation factor 1-alpha 2, NF-kappa-B-activating protein, Phosphatidylinositol-4 phosphate 5-kinase type II alpha, Caesin kinase 2A1, Phosphatidylinositol-4 phosphate 5-kinase type II gamma, Phosphatidylinositol-4 phosphate 5-kinase type II beta, Hypothetical protein FLJ32377, RNF187 protein, Small acididc protein, Multiple myeloma tumor-associated protein 2, Hepatoma derived growth factor 2, UBF and SC-35.

A fragment of a protein involved in RNA metabolism, processing or splicing, preferably a splicing regulatory protein, may be used in a method of the invention. The fragment contains a K residue that can be hydroxylated by Jmjd6. The fragment is typically a peptide of at least 11 amino acids in length, such as a peptide of at least 12, 13, 14, 15 or 16 amino acids in length. The fragment may be rich in R and S residues. The fragment typically comprises two, three or more RS motifs and may comprise a RS domain as described above. Thus, the method of the invention may utilise a fragment of the splicing regulatory protein that is rich in arginine and serine residues and comprises at least one lysine residue.

The fragment may be modified by the substitution or deletion of one or more amino acid residue present in the native protein sequence. One or more amino acid may also be added to the sequence of the peptide fragment, preferably at one or both ends of the peptide. Examples of suitable fragments include peptides of at least 11 amino acids comprising the amino acid sequence NPKXSXSXEHR, and peptides of at least 12 amino acids comprising the amino acid sequence NPKXSXSXEHRR, wherein X is K or R. Further examples of suitable fragments include peptides of at least 16 amino acids comprising the amino acid sequence SHSRSRSRDRKRRSRS or peptides of at least 17 amino acids comprising the amino acid sequence the amino acid sequence RDKENRHRICRSHSRSRS.

The protein or fragment useful as a Jmjd6 substrate in a method of the invention may be methylated at one or more residue. For example, one or more R residue may be mono-, di- or tri- methylated. K residues may also be methylated within the substrate protein or peptide, provided that at least one K residue that is unmethylated so that hydroxylation by Jmjd6 is not inhibited. Examples of specific peptides that are hydroxylated by Jmjd6 are shown in Tables 3 and 4. In a preferred embodiment of the invention, the splicing regulatory protein is the splicing factor U2AF 65 kDa subunit (U2AF65), Luc7-like2 or cisplatin resistance-associated over-expressed protein (CROP), or a fragment of any thereof comprising a lysine residue.

The K residue in the Jmjd6 substrate may be modified, i.e. it may be an analogue of K. The modification(s) on the K residue are modifications that do not prevent hydroxylation by Jmjd6. Modification of the ε-amino acid group blocks hydroxylation and so the K residue is preferably not modified by methylation. Hydroxylation of K residues as catalysed by Jmjd6 may affect modification of the ε-amino group, for example, by acetylation, sumoylation, ubiquitination or transamination. Transamination may be followed by a further reaction of the resulting aldehyde group. Accordingly, the substrate may comprise a K residue that is acetylated, sumoylated, ubiquinated or transaminated. The modification may occur before or after hydroxylation of the substrate by Jmjd6.

The Jmjd6 polypeptide may comprise the sequence shown in SEQ ID NO: 1, or may be a fragment or variant of SEQ ID NO: 1 having lysyl hydroxylase activity. Fragments of Jmjd6 are described in more detail below. The Jmjd6 polypeptide may have an amino acid sequence having at least about 60% sequence identity, for example at least about 70% sequence identity, with SEQ ID NO: 1 over its entire length or over an active fragment thereof (such as SEQ ID NO: 2 or 3), typically greater than about 80% or 90%, such as about 95% or about 99% sequence identity.

Sequence identity may be calculated using any suitable algorithm. For example, the UWGCG Package provides the BESTFIT program can be used to infer homology (for example used on its default settings) (Devereux et al. (1984) Nucleic Acids Research 12, p387-395). The PILEUP and BLAST algorithms can be used to infer homology or line up sequences (typically on their default settings), for example as described in Latched (1993) J. Mol. Evol 36:290-300; Latched et al. (1990) J. Mol. Biol. 215:403-10.

The Jmjd6 polypeptide may be a polypeptide encoded by any naturally occurring *Jmjd6* gene. The naturally occurring *Jmjd6* gene may comprise the sequence shown in SEQ ID NO: 1 or may be a variant of SEQ ID NO: 1. Such variants may include allelic variants and the deletion, modification or addition of single amino acids or groups of amino acids within the protein sequence, as long as the polypeptide retains lysyl hydroxylase activity.

Amino acid substitutions of SEQ ID NO: 1, or of a fragment thereof (such as SEQ ID NO: 2 or 3) may be made, for example from about 1, 2 or 3 to about 10, 20 or 30 substitutions. Conservative substitutions may be made, for example according to the following Table. Amino acids in the same block in the second column and preferably in the same line in the third column may be substituted for each other.

| | | |
|---|---|---|
| ALIPHATIC | Non-polar | GAP |
| | | I L V |
| | Polar-uncharged | CSTM |
| | | NQ |
| | Polar-charged | DE |
| | | KR |
| AROMATIC | | HFWY |

Variant polypeptides within the scope of the invention may be generated by any suitable method, for example by gene shuffling techniques.

The present invention also includes use of active portions, fragments, derivatives and functional mimetic of the polypeptides of the invention. An "active portion" of a polypeptide means a peptide which is less than said full-length polypeptide, but which retains lysyl hydroxylase activity. An active fragment of Jmjd6 may typically be identified by monitoring for 2-OG oxygenase activity as described in more detail below. Such an active fragment may be included as part of a fusion protein.

The fragment may have up to about 60, 70, 80, 100, 150, 200, 300, 350 or 400 amino acids.

The fragment may comprise any region from about amino acid 1 to about 403 of the amino acid sequence shown in SEQ ID NO: 1, such as from amino acid 2, 3, 4, 5 or about 10 to about amino acid 330, 340, 350, 360, 370, 380, 390 or 400. Useful fragments include N-terminal truncated fragments i.e., fragments comprising an N-terminal deletion, such as fragments comprising residues 10 to 403, 20 to 403 or 25 to 403 of the amino acid sequence shown in SEQ ID NO: 1, and specific examples include fragments comprising residues 5 to 403, 8 to 403, 11 to 403, 14 to 403, 26 to 403, 49 to 403, 88 to 403 or 132 to 403. In one embodiment, the fragment comprises residues 26 to 48 to enable the Jmjd6 protein to homo-oligomerise. Useful fragments also include fragments comprising C-terminal truncations such as fragments comprising residues 1 to 390, 1 to 380 or 1 to 350 of the amino acid sequence shown in SEQ ID NO: 1, and specific examples include fragments comprising residues 1 to 221, 1 to 314, 1 to 337, 1 to 362 and 1 to 379. In one embodiment, the fragment comprises residues 338 to 362 (preferably comprising the poly-S region) to enable the Jmjd6 protein to preferentially localise to the nucleoplasm. Alternatively, the fragment may not comprise residues 338 to 362 (preferably not comprising the poly-S region) to enable the Jmjd6 protein to preferentially localise to the nucleolus. Useful fragments also include fragments comprising both N-terminal and C-terminal truncations, such as fragment comprising residues 10 to 390,20 to 380 or 25 to 350 of the amino acid sequence shown in SEQ ID NO: 1. If Jmjd6 is to be contacted by U2AF-65 then, preferentially, Jmjd6 should not feature an N-terminal deletion of greater than 4 amino acids or C-terminal deletion of greater than 24 amino acids, nor should it contain a H187A mutation or contain H187A and D189A mutations. Other examples of specific truncated fragments that may be used in the invention are shown in SEQ ID NOs: 2 and 3 (residues 1 to 343 and residues 25 to 338, respectively). Other suitable fragments may readily be identified, for example by comparing the Jmjd6 amino acid sequence to the amino acid sequence of one or more known 2-OG dependent oxygenase and identifying which regions are homologous to regions having catalytic activity. The regions having catalytic activity are typically included in the active fragments. Such fragments can be used to construct chimeric molecules.

Fragments of any Jmj d6 polypeptide having at least about 60%, such as at least about 70%, 80%, 90%, 95% or 99% sequence identity to the amino acid sequence shown in SEQ ID NO: 1, which fragments have lysyl hydroxylase activity may also be used in an assay of the invention and are encompassed within the term "Jmjd6 polypeptide" used herein.

The Jmjd6 polypeptide may comprise one or more particular site directed mutations, such as mutations in the active site and/or the AT-hook region, preferably selected from H187A and D189A (active site mutations, ASM1 and ASM2 respectively) and G304A, R305A and P306A (AT-hook mutations). Preferably, the Jmjd6 polypeptide comprises H187A, or H187A and D189A, or G304A, R305A and P306A. None of these combinations of mutations affect oligomerisation. As mentioned above, Jmjd6 polypeptides comprising H187A, or H187A and D189A, do not interact with U2AF-65. Mutations to the active site can alter the Fe(II) binding site and can therefore reduce or abolish the catalytic activity of the Jmjd6 polypeptide. Jmjd6 polypeptides comprising H187A and/or D189A are expected to have reduced or abolished activity and can therefore be used to screen for molecules that interact with or bind to Jmjd6 but are not necessarily hydroxylated, or to screen for molecules that modulate Jmjd6 activity, such as inhibitors or activators, preferably activators.

In one embodiment, therefore, the invention provides a method for assaying for molecules that bind Jmjd6, the method comprising contacting a test molecule, preferably a splicing regulatory protein, or a fragment or variant thereof comprising a lysine residue, with a Jmjd6 polypeptide comprising H187A and/or D189A, and determining whether the test molecule binds Jmjd6. Binding of a molecule may be assessed using standard techniques in the art.

In a further embodiment the invention provides a method of assaying for molecules that modulate Jmjd6 activity, preferably molecules that increase Jmjd6 activty (i.e. activators), the method comprising contacting a splicing regulatory protein, or a fragment or variant thereof comprising a lysine residue, with a Jmjd6 polypeptide comprising H187A and/or D189A in the presence of a test molecule and determining whether the test molecule is a modulator (preferably an activator) of Jmjd6 activity. A modulator that increases the activity of or restores the activity of the mutated Jmjd6 polypeptide is an activator. A modulator that further decreases the activity of or abolishes the activity of the mutated Jmjd6 polypeptide is an inhibitor. Whether or not the test agent modulates the activity of the mutated Jmjd6 polypeptide can be determined as discussed below.

The Jmjd6 polypeptides may be synthetically prepared. The polypeptides may be chemically or biochemically modified, e.g. post-translationally modified. For example, they may be glycosylated or comprise modified amino acid residues. They may also be modified by the addition of histidine residues (typically six), or other sequence tags such as a maltose binding protein tag or intein tag, to assist their purification or by the addition of a nuclear localisation sequence to promote translocation to the nucleus or mitochondria, and or by post translational modification including hydroxylation or phosphorylation. Polypeptides of the invention may be GST or other suitable fusion polypeptides. The Jmjd6 polypeptide may also be modified by addition of fluorescent tags (such as green or yellow fluorescent protein) to enable visualisation within cells or organelles or to aid purification of the protein or cells expressing Jmjd6. Such modified polypeptides fall within the scope of the term "Jmjd6 polypeptide".

The Jmjd6 polypeptide of the invention may be present in a partially purified or in a substantially isolated form. The polypeptide may be mixed with carriers or diluents, which will not interfere with its intended use and still be regarded as substantially isolated. The polypeptide may also be in a substantially purified form, in which case it will generally comprise at least about 90%, e.g. at least about 95%, 98% or 99%, of the proteins, polynucleotides, cells or dry mass of the preparation.

The Jmjd6 polypeptide used in a method of the invention may be recombinant Jmjd6 or naturally occurring Jmjd6. Naturally occurring Jmjd6 may be obtained from any organism that produces a Jmjd6 polypeptide. Preferably, recombinant Jmjd6 is used especially where Jmjd6 is required for purposes requiring large (> 1 mg) amounts of protein such as for biophysical assays or for high throughput analyses. Recombinant Jmjd6 may be produced using standard expression vectors that comprise nucleotide sequences encoding Jmjd6. Such expression vectors are routinely constructed in the art of molecular biology and may for example involve the use of plasmid DNA and appropriate initiators, promoters, enhancers and other elements, such as for example polyadenylation signals which may be necessary, and which are positioned in the correct orientation, in order to allow for protein expression. Other suitable vectors would be apparent to persons skilled in the art. By way of further example in this regard we refer to Sambrook *et al.* 1989.

The Jmjd6 polypeptide may be present in a cell. For example, methods of the invention may utilise cells that have been modified to express a Jmjd6 polypeptide as defined herein. The Jmjd6 may also be present in a cell extract or in a partially or substantially purified form.

A purified Jmjd6 polypeptide may be obtained by introducing an expression vector comprising a polynucleotide encoding a Jmjd6 polypeptide into a host cell.

Expression vectors are routinely constructed in the art and may for example involve the use of plasmid DNA and appropriate initiators, promoters, enhancers and other elements, such as for example polyadenylation signals which may be necessary and which are positioned in the correct orientation in order to allow full protein expression. Suitable vectors would be very readily apparent to those of skill in the art. Promoter sequences may be inducible or constitutive promoters depending on the selected assay format. The promoter may be tissue specific. Thus the coding sequence in the vector is operably linked to such elements so that they provide for expression of the coding sequence (typically in a cell). The term "operably linked" refers to a juxtaposition wherein the components described are in a relationship permitting them to function in their intended manner.

The vector may be, for example, a plasmid, virus or baculovirus vector. The vector is typically adapted to be used in a bacterial cell, such as *E*. *coli.* The vector may have an origin of replication. The vector may comprise one or more selectable marker genes, for example an ampicillin resistance gene in the case of a bacterial plasmid or a resistance gene for a fungal vector. Vectors may be used to transfect or transform a host cell, for example, a bacterial host cell, fungal host cell, an insect host cell, a mammalian, e.g. human host cell or a baculovirus host cell. The bacterial host cell is preferably a strain of *E. coli,* for example BL21 (DE3).

Methods for introducing polypeptides and vectors into host cells are well known in the art, and include electroporation and heat shock techniques without limitation. Expression of the truncated polypeptide may then be achieved by culturing the host cells.

The Jmjd6 polypeptide may be purified by lysing the host cells and extracting Jmjd6 from the soluble fraction, for example by affinity purification, such as via an affinity tag fused to the truncated Jmjd6 polypeptide. Jmjd6 polypeptides may be purified by standard techniques known in the art. For example, where the polypeptide comprises a His tag, it may be purified using a his-binding resin by following the manufacturer's instructions (e.g. Novagen) or by other means such as ion exchange chromatography.

The method of the invention may be used to identify a modulator of Jmjd6 activity. The assay may be carried out in the presence of a test agent to determine whether the test agent is a modulator of Jmjd6 activity. Any suitable assay may be carried out to identify modulators of Jmjd6 lysyl hydroxylase activity. A number of different examples of suitable assays are described below. Assays of the invention may be used to identify an agent which modulates, such as inhibits or activates, Jmjd6 lysyl hydroxylase activity.

In a method of the invention Jmjd6 activity may be assayed by monitoring oxygenase activity of a Jmjd6 polypeptide in the presence of substrate, wherein the substrate is a protein expression regulatory protein, such as a splicing regulatory protein. The substrate and Jmjd6 polypeptide, and optionally the test agent, are typically contacted under conditions suitable for oxygenase (lysyl hydroxylase) activity.

Suitable co-substrates include oxygen, for example, dioxygen, and 2-oxoacids such as 2-oxogluterate (2-OG) or 2-OG analogues. Preferably, the co-substrate is 2-OG. In addition to oxygen or a 2-oxoacid, a reducing agent, such as ascorbate may also be used as a co-substrate. Thus, in a method according to the invention, the splicing regulatory protein or fragment thereof and Jmj d6 polypeptide are contacted in the presence of Fe(II), oxygen and 2-oxoglutarate and optionally in the presence of a reducing agent.

Hydroxylation of the substrate may be assayed directly or indirectly. Such assays may employ techniques such as chromatography, NMR, MS or fluorescence spectroscopy. The co-substrate may be modified, e.g. 2-OG, consumed, e.g. oxygen or ascorbate, or produced, e.g. succinate or carbon dioxide, by Jmjd6.

In an assay to identify a modulator of Jmjd6 activity, the components of the assay are contacted under conditions in which Jmjd6 has lysyl hydroxylase activity both in the absence of the test agent and in the presence of the test agent so that the effect of the test agent on Jmjd6 activity may be determined. The assay may also be used to detect agents that increase or decrease the activity of Jmjd6 activity by assaying for increases or decreases in activity. Suitable assays have been described in the art for other 2-OG dependent oxygenases.

Assays of the present invention may be used to identify inhibitors of oxygenase activity and are thus preferably carried out under conditions under which Jmjd6 is active as an oxygenase (a lysyl hydroxylase) in the absence of the test agent. The Jmjd6 oxygenase activity in the presence of the test agent is compared to Jmjd6 oxygenase activity in the absence of the test substance to determine whether the test substance is an inhibitor of Jmjd6 oxygenase activity. In the alternative, the assays may be used to look for promoters of Jmjd6 oxygenase activity, for example, by looking for increased conversion of co-substrate and/or hydroxylation of substrates compared to assays carried out in the absence of a test substance. The assays may also be carried out under conditions in which oxygenase activity is reduced or absent, such as under hypoxic conditions, and the presence of or increased activity could be monitored under such conditions.

In medicinal applications, for example, it is often advantageous to modulate oxygenase activity of a single enzyme or group of enzymes. The assays of the invention may also be used to identify inhibitors or activators that are specific for Jmjd6 and which do not have activity or are less active with other 2-OG oxygenases. Conversely, the assays of the invention may be used to identify inhibitors or activators specific for one or more 2-OG dependent oxygenase which do not inhibit Jmjd6 activity. 2-OG oxygenases that may be tested in such a method of the invention include, but are not limited to: lysyl, prolyl, asparaginyl and arginyl demethylases, hypoxia inducible factor (HIF) asparaginyl or prolyl hydroxylases, including FIH, PHD1, PHD2 and PHD3, AlkB, ABH1, ABH2, ABH3, procollagen prolyl and lysyl hydroxylases, methyl lysine demethylases, Mina53, the fat mass and obesity protein, the epidermal growth factor hydroxylases and other 2-OG oxygenases that have been characterized as Jmj domain proteins according to the SMART database including, but not limited to lysyl demethylases.

The present invention also provides a method for identifying a selective inhibitor of Jmjd6, or an inhibitor that is selective for another 2OG-oxygenase over Jmjd6. This method comprises: (i) contacting a protein involved in RNA metabolism, processing or splicing, or fragment thereof comprising a lysine residue, with a Jmjd6 polypeptide in the presence of a test agent and determining whether the protein or fragment thereof is hydroxylated; (ii) determining whether the test agent modulates activity of a 2-OG dependent oxygenase other than Jmjd6, thereby determining whether the test agent selectively modulates Jmjd6 activity or selectively modulates activity of the 2-OG dependent oxygenase other than Jmjd6.

Oxygenase acvtivity of the 2-oxoglutarate dependent oxygenase other than Jmjd6 may be determined by contacting a substrate of the 2-OG dependent oxygenase with the 2-OG dependent oxygenase in the presence of a test agent and determining whether the substrate is hydroxylated or demethylated. In an assay to identify a selective inhibitor of Jmjd6, or another oxygenase, different substrates may be used for Jmjd6 and for the other oxygenase(s).

Alternatively, oxygenase activity of the 2-OG dependent oxygenase other than Jmjd6 may be determined in the absence of a prime substrate (i.e, a non 2-OG substrate). This enables selective inhibitors to be identified when the prime substrate of one or more of the enzymes being tested is unknown. In this embodiment, generally it will be one or more of the enzymes that it is wished not to inhibit that is an enzyme that has an unknown substrate. The effect of a test agent on activity of an oxygenase may be determined in the absence of a substrate by determining whether or not the test agent affects, for example inhibits or stimulates, the rate of turnover of 2-OG by the oxygenase.

Thus, the invention also provides methods for screening for compounds that do not inhibit Jmjd6. Such compounds are of use with respect to developing inhibitors that are selective for 2-OG oxygenases other than Jmjd6, such as, for example, the HIF prolyl and asparaginyl hydroxylases or the 2-OG dependent histone lysyl demethylases.

The assays of the invention may also be used to identify inhibitors or activators, which are specific for Jmjd6 activity at a particular substrate or residue within a substrate.

Such selectivity screens may be used to identify selective inhibitors of Jmjd6 or selective inhibitors of other enzymes, i.e. inhibitors that are more potent inhibitors of Jmjd6 activity than of activity of the other enzyme or inhibitors that are less potent inhibitors of Jmjd6 activity than of activity of the other enzyme. Where the inhibitor is a selective inhibitor of Jmjd6 activity it may have no effect on the activity of the other enzyme or may exhibit only a low level of inhibition, such as less than about 50% inhibition on activity of the other enzyme. Where the inhibitor is a selective inhibitor of the activity of the enzyme other than Jmjd6, it may have no effect on the activity of Jmjd6 or may exhibit only a low level of inhibition, such as less than about 50% inhibition of Jmjd6 activity.

The selectivity screens may be carried out with purified enzymes, partially purified enzymes (such as in crude cell lysates) or in cells.

The invention provides for the use of selective inhibitors in the manufacture of a medicament for the treatment of a condition associated with altered, i.e. enhanced or reduced, 2-OG dependent oxygenase activity, such as Jmjd6 oxygenase activity.

The precise format of any of the assay or screening methods of the present invention may be varied by those of skill in the art using routine skill and knowledge. The skilled person is well aware of the need to additionally employ appropriate controlled experiments. The assays of the present invention may involve monitoring for hydroxylation of the substrate, monitoring for the utilisation of substrates and co-substrates, monitoring for the production of the expected products between the enzyme and its substrate. Assay methods of the present invention may also involve screening for the direct interaction between components in the system. Alternatively, assays may be carried out which monitor for downstream effects mediated by the substrate, such as substrate mediated transcription using suitable reporter constructs or by monitoring for the upregulation of genes or alterations in the expression patterns of genes known to be regulated directly or indirectly by the substrate.

Various methods for determining oxygenase activity either directly or indirectly are known in the art. Any suitable method may be used for determining 2-OG dependent oxygenase activity of Jmjd6 such as by substrate or co-substrate utilisation, product appearance such as peptide hydroxylation (or demethylation for some 2-OG oxygenases) or down-stream effects mediated by hydroxylated (or demethylated or non-hydroxylated products for some 2-OG oxygenases).

The substrate, enzyme and potential inhibitor compound may be incubated together under conditions which, in the absence of inhibitor provide for hydroxylation (or demethylation for some 2-OG oxygenases) of the substrate, and the effect of the inhibitor may be determined by determining hydroxylation (or demethylation for some 2-OG oxygenases) of the substrate. This may be accomplished by any suitable means. Small polypeptide or polynucleotide substrates may be recovered and subjected to physical analysis, such as mass spectrometry, radiography or chromatography, or to functional analysis. Such methods are known as such in the art and may be practiced using routine skill and knowledge. For example, the LC-MS assay described in the Examples may be used. Determination may be quantitative or qualitative. In both cases, but particularly in the latter, qualitative determination may be carried out in comparison to a suitable control, e.g. a substrate incubated without the potential inhibitor.

In alternative embodiments, reporter constructs may be provided in which promoters mediated by a substrate are provided operably linked to a reporter gene. Any suitable reporter gene could be used, such as for example enzymes which may then be used in colorometric, fluorometric, fluorescence resonance or spectrometric assays.

In the assay methods described herein, typically the Jmjd6 polypeptide and the substrate are contacted in the presence of a co-substrate, such as oxygen and/or a 2-oxoacid, such as 2-OG, and/or dioxygen. Hydroxylase activity may be determined by determining turnover of one or more of the co-substrates, such as oxygen, 2-OG and/or ascorbate. This may be achieved by determining the presence and/or amount of reaction products, such as hydroxylated substrate, carbon dioxide or succinic acid. The amount of product may be determined relative to the amount of substrate. For example, in such embodiments the product measured may be hydroxylated peptide or protein. For example, the extent of hydroxylation may be determined by measuring the amount of hydroxylated peptide/protein, succinate, carbon dioxide, or formaldehyde generated in the reaction, or by measuring the depletion of 2-OG or dioxygen. Methods for monitoring each of these are known in the scientific literature, for example in Myllyharju et al. (1991) EMBO J. 16(6): 1173-1180 or as in Cunliffe et al. (1986) Biochem. J. 240: 617-619.

Unused 2-OG may be derivatised by chemical reagents, exemplified by but not limited to hydrazine derivatives and *ortho*-phenylene diamine derivatives, to give indicative chromophores or fluorophores that can be quantified and used to indicate the extent of hydroxylation of the substrate. Suitable methods are described in McNeill et al (2005) (Anal. Biochem. 366:125-131). Dissolved oxygen electrodes, exemplified by but not limited to a "Clarke-type" electrode or an electrode that uses fluorescence quenching, may be used to follow the consumption of oxygen in an assay mixture, which can then be used to indicate the extent of hydroxylation of the test polypeptide in an analogous manner to the above.

The fluorescent product of the reaction of *ortho*-phenylenediamine (OPD) with the α-ketoacid motif of 2-OG is 3-(2-Carboxyethyl)-2(1*H*)-quinoxalinone. This fluorescent product can be readily detected by standard equipment such as that manufactured by for example Molecular Devices, Tecan, BMG Labtechnologies, Jasco and Perkin Elmer and there is extensive precedent demonstrating that the production of fluorescent products can be used in high-throughput screens.

The fluorescent product is generally detected with the excitation filter set as from about 300nm to about 400nm, preferably from about 335 to about 345 nm, most preferably at about 340nm. The emission filter is generally at from about 400 to about 450nm, preferably from about 415 to about 425nm, most preferably at about 420nm.

This assay procedure lends itself to high-throughput formats, such as multi-well plate formats e.g. 96-, 384-, or 1536-well plate formats.

Further, the nature of the fluorescent product can be tuned by modifying the nature of the derivatisation reagent used. For example, the sensitivity of the method may be increased by using either 1,2-dimethoxy-4,5-diaminobenzene, or 1,2-methylenedioxy-4,5-diaminobenzene.

The precise format of any of the screening or assay methods of the present invention may be varied by those of skill in the art using routine skill and knowledge. The skilled person is well aware of the need to additionally employ appropriate control experiments. Activity is measured by derivatisation of 2-OG with OPD or other aromatic diamines, such as 1,2-dimethoxy-4,5-diaminobenzene or 1,2-methylenedioxy-4,5-diaminobenzene, such that the derivative gives improved sensitivity compared to use of OPD (Mühling et al. Journal of Chromatography B (2003) 383-392, Nakamura et al. Chem. Pharm Bull. (1987) 687-692).

The assay is carried out under conditions suitable for hydroxylation/oxidation of the substrate by the oxidase. Accordingly, 2-OG is present in the assay. The assay mixture may also contain iron, preferably ferrous iron.

Other components may be added to the assay mixtures. For example, a reducing agent such as ascorbate, a thiol such as dithiothrietol (DDT), β-mercaptoethanol, tris(2-carboxyethyl)phosphine hydrochloride (TCEP), N-acetylcysteine or phenol may be added to the assay to help maintain enzyme structure and/or catalase may be added to destroy any H₂O₂ that might be produced. However, the assay will work in the absence of a reducing agent or catalase.

Assays are typically carried out at a temperature of from about 25°C to about 40°C, for example at a temperature of from about 30°C to about 39°C, or from about 35°C to about 38°C or about 37°C. The pH of the assay mixture is typically between about pH 7 to about pH 9, for example from about pH 7.5 to about pH 8. Suitable buffers, such as Tris or HEPES, may be used to maintain the pH of the assay mixture.

Typically, assays are carried out under normoxic conditions. The assay may also be carried out under conditions in which hydroxylation or oxidation is reduced or absent, such as under hypoxic conditions, in order to detect modulation of oxygenase activity by an agent which enhances hydroxylation/oxidation.

Alternatively, the end-point determination may be based on conversion of the substrate or substrate fragments (including synthetic and recombinant peptides or nucleic acids) derived from the polypeptide or nucleic acid substrate into detectable products. Substrates may be modified to facilitate the assays so that they can be rapidly carried out and may be suitable for high throughput screening.

For example, reverse phase HPLC (C-4 octadecylsilane column), as exemplified herein, may be used to separate starting synthetic peptide substrates for subtraters from the products. Modifications of this assay or alternative assays for oxygenase activity may employ, for example, mass spectrometric, spectroscopic, and/or fluorescence techniques as are well known in the art (Masimirembwa C. et al. Combinatorial Chemistry & High Throughput Screening (2001) 4 (3) 245-263, Owicki J. (2000) J. Biomol. Screen. 5 (5) 297-305, Gershkovich A et al. (1996) J. Biochem. & Biophys. Meths. 33 (3) 135-162, Kraaft G. et al. (1994) Meths. Enzymol. 241 70-86). Fluorescent techniques may employ versions of the substrate modified in such as way as to carry out or optimise spectroscopic or fluorescence assays.

Binding of a molecule, such as an antibody, which discriminates between the hydroxylated and non-hydroxylated forms of a peptide or protein may be assessed using any technique available to those skilled in the art, which may involve determination of the presence of a suitable label.

Assay methods of the present invention may also take the form of *an in vivo* assay or an assay carried out on *ex vivo* cells from an animal, such as a mammal (including human) or an insect. The assay may be performed in a cell line such as a yeast or bacterial strain or an insect or mammalian cell line in which the relevant polypeptides or peptides are expressed from one or more vectors introduced into the cell. Alternatively, the assay may be carried out on a mammalian cell that expresses endogenous Jmjd6 or in which Jmjd6 is over expressed.

The present invention further provides a method for identifying a modulator of RNA splicing, the method comprising contacting a cell which expresses Jmjd6 with a test agent and determining whether the test agent modulates Jmjd6 regulation of RNA splicing.

In one embodiment Jmjd6 may be over-expressed in the cell. Jmjd6 may be over-expressed in a cell *in vitro* or *in vivo* by any suitable method, typically by introducing an expression vector encoding a Jmjd6 polypeptide into the cell. RNA splicing may be monitored in the cell over-expressing Jmjd6 and compared to RNA splicing in a control cell that does not over-express Jmjd6. The cell over-expressing Jmjd6 may be contacted with a test agent and RNA splicing may be monitored in the presence of the test agent. By comparing the RNA splicing observed in the presence and absence of the test agent and in the presence and absence of Jmjd6 over-expression, it may determined whether the test agent modulates Jmjd6-mediated regulation of RNA splicing.

In another embodiment, Jmjd6 may be under-expressed in the cell. Jmjd6 may be under-expressed in a cell *in vitro* or *in vivo* by any suitable method, for example by using RNAi technology to knock down the Jmjd6 protein. RNA splicing may be monitored in the cell under-expressing Jmjd6 and compared to RNA splicing in a control cell that does not under-express Jmjd6. The cell under-expressing Jmjd6 may be contacted with a test agent and RNA splicing may be monitored in the presence of the test agent. By comparing the RNA splicing observed in the presence and absence of the test agent and in the presence and absence of Jmjd6 under-expression, it may determined whether the test agent modulates Jmjd6-mediated regulation of RNA splicing.

Methods for monitoring RNA splicing are well known in the art. For example, RNA splicing may be monitored using a reporter construct. Thus, in a method for identifying a modulator of RNA splicing acoording to the invention, the cell may comprise a RNA splicing reporter construct and the method may comprise determining whether Jmjd6-mediated regulation of RNA splicing of the reporter construct is modulated by the test agent.

An example of a construct for monitoring regulation of alternative splicing is the α-tropomyosin (α -TM) gene construct. This construct recapitulates splicing regulation of the α-TM gene and contains exons 1, 3 and 4 surrounded by their intronic regulatory sequences (Figure 16B). Although the α-TM minigene lacks the mutually exclusive exon 2, this does not interfere with the splicing regulation of exon 3. Splicing of α-TM minigene construct generates two mRNA isoforms: 134 and 14. Repression of exon 3 splicing is mediated by negative regulatory elements surrounding this exon: the polypyrimidine tract (PPT), Upstream and Downstream Regulatory Elements (URE and DRE).

The amount of both α-TM spliced isoforms can be determined by RT-PCR analysis in Jmjd6-knocked down and control cells as well as in Jmjd6-knocked down cells contacted with a test agent, transfected (either transiently or stably) with the α-TM minigene construct. A change in the amount of each isoform in the presence of the test agent compared to in the absence of the test agent indicates that the test agant is a modulator of Jmjd6-mediated regulation of RNA splicing.

Another example of a suitable RNA splicing reported construct is described in Nasim et al (2002) Nucleic Acids Research 30(20): e109. This reporter contains beta-galactosidase and luciferase reporter genes separated by an intron. Beta-galactosidase is always expressed and luciferase is expressed only when the intron is spliced out.

Agents, which may be screened using the assay methods described herein, may be natural or synthetic chemical compounds used in drug screening programmes. Extracts of plants, microbes or other organisms, which contain several, characterised or uncharacterised components may also be used.

Combinatorial library technology (including solid phase synthesis and parallel synthesis methodologies) can provide an efficient way of testing a potentially vast number of different substances for ability to modulate an interaction. Such libraries and their use are known in the art, for all manner of natural products, small molecules and peptides, among others. The use of peptide libraries may be preferred in certain circumstances. Various commercial libraries of compounds are also available. There are computational methods for screening these libraries (processes sometimes referred to as virtual screening) that can identify lead structures for inhibition.

Potential inhibitor compounds (i.e. antagonists) may be polypeptides, peptides, small molecules such as molecules from commercially available libraries, including combinatorial libraries, or the like. The peptide may be a cyclic peptide.

Small molecule compounds, which may be used, include 2-OG analogues, or substrate analogues, which inhibit the action of the enzyme. Small molecule compounds, and other types of compound, that may be used include all known 2-OG oxygenase inhibitors such as those already known to inhibit HIF hydroxylases (see for example WO03/080566, WO02/074981, WO2007/146483, WO2007136990, WO2007/103905, W02007/150011, US2007/0299086, US2007/0249605 and US2007/0213335) and procollagen prolyl hydroxylases.

Potential promoting agents may be screened from a wide variety of sources, particularly from libraries of small compounds, which are commercially available. Oxygen-containing compounds may be included in candidate compounds to be screened, for example 2-OG analogues.

Since naturally occurring compounds, including TCA cycle intermediates such as fumarate and succinate, are known inhibitors of 2-OG oxygenases they may inhibit Jmjd6, possibly in a manner that is of physiological relevance, including in some cancers where fumarate is known to be upregulated as a consequence of the Warburg effect.

A test compound which increases, potentiates, stimulates, disrupts, reduces, interferes with or wholly or partially abolishes hydroxylation of the substrate and which may thereby modulate activity, may be identified and/or obtained using the assay methods described herein.

Agents which increase or potentiate hydroxylation (i.e. agonists), may be identified and/or obtained under conditions which, in the absence of a positively-testing agent, limit or prevent hydroxylation. Such agents may be used to potentiate, increase, enhance or stimulate the oxygenase activity of Jmjd6.

An agent or compound identified by a screening method of the invention to be a modulator of Jmjd6 oxygenase activity may be a substance which inhibits or reduces, increases or potentiates the activity of Jmjd6.

The test agent may compete with 2-OG or a Jmjd6 substrate at the Jmjd6 active site and/or binds to the active site of Jmjd6 or to metal at the Jmjd6 active site. The test agent may comprise a metal ion such as, but not limited to, iron (II), iron (III), manganese, cobalt, zinc or nickel ions. Alternatively, the mode of inhibition may be via competition with the substrate or an allosteric interaction.

The test agent may be a reducing agent. Reducing agents typically act as activators of 2-OG oxygenase activity, typically *in vitro.* An activator of oxygenase activity may be any species that increases oxygenase activity of a Jmjd6 polypeptide either *in vitro* or *in vivo.* Reducing agents that may be used include ascorbate and analogues of ascorbate and reducing agents of the thiol chemical families, such as dithiothreitol or phosphine (e.g. triscarboxyethylphosphine).

Following identification of a modulator, the substance may be purified and/or investigated further (e.g. modified) and/or manufactured. A modulator may be used to obtain peptidyl or non-peptidyl mimetics, e.g. by methods well known to those skilled in the art and discussed herein. A modulator may be modified, for example to increase selectively, as described herein. It may be used in a therapeutic context as discussed below.

For therapeutic treatment, the modulator may be alone or used in combination with any other therapeutically active substance or treatment.

The compounds which are acids can be present in the form of salts, such as sodium salts. The compounds may also be present in the form of derivatives such as the dimethyl ester, diethyl ester, monoethyl ester or di- or mono-amide. In certain instances these derivatives may be preferred, for example when inhibition of the enzyme within a cell of an organism is required.

Compounds which modulate 2-OG oxygenases may be useful as agents, for example, in the treatment of disorders as described herein, or may be used as test substances in an assay of the invention. The test compound may be known to act as an inhibitor of a 2-OG oxygenase other than Jmjd6. For example, the test agent may be an inhibitor of procollagen prolyl hydroxylase, hypoxia inducible factor, prolyl and asparaginyl hydroxylases, collagen prolyl hydroxylase, gibberellin C-20 oxidase, a nucleic acid demethylase such as AlkB or a human AlkB homologue, a protein demethylase, such as a tri-, di-, mono-methyl lysine or arginine residue demethylase, another human or animal 2OG oxygenase involved in metabolism or regulation, or a plant 2-OG hydroxylase. Many inhibitors of 2OG oxygenases are known in particular for human prolyl hydroxylases. N-oxaloglycine and its derivatives are suitable examples. Glycine or alanine derivatives and 2-oxoacid analogues may also be used.

Compounds which modulate 2-OG oxygenases, and families of such compounds, are known in the art, for example in Aoyagi et al. (2002) Hepatology Research 23 (1): 1-6, Aoyagi et al. (2003) Free Radical Biology and Medicine 35:410 Suppl. 1, Philipp et al. (2002) Circulation 106 (19): 1344 Suppl. S, Ivan et al. (2002) PNAS USA 99 (21): 13459-13464, Nwogu et al. (2001) Circulation 104 (18): 2216-2221, Myllyharju and Kivirikko (2001) Ann Med 33 (1): 7-21, Ohta et al. (1984) Chemical and Pharm Bulletin 32 (11): 4350-4359, Franklin et al. (2001) Biochem J. 353: 333-338, Franklin (1997) Int J. Biochem Cell Biol 29 (1): 79-89, Dowell et al. (1993) Eur J Med Chem 28 (6): 513-516, Baader et al. (1994) Biochem J. 300: 525-530, Baader et al. (1994) Eur J Clin Chem and Clin Biol 32 (7): 515-520, Bickel et al. (1998) Hepatology 28 (2): 404-411, Bickel et al. (1991) J. Hepatology 13: S26-S34 Suppl. 3, US 6,200,974, US 5,916,898, US Patent Applications 2003-0176317, 2003-0153503 and 2004-0053977, WO 02/074981, WO 03/080566, WO 04/035812, Cunliffe et al. (1992) J. Med. Chem. 35:2652-2658, Higashide et al. (1995) J. Antibiotics 38:285-295, Cunliffe et al. (1986) Biochem. J. 239(2):311-315, Franklin et al. (1989) Biochem. J. 261(1):127-130, Friedman et al. (2000) PNAS USA 97(9):4736-4741, Wu et al. (1999) J. Am. Chem. Soc. 121(3): 587-588, DE-A-3818850, Wang et al. (2001) Biochemistry US:15676-15683 and Lerner et al. (2001) Angew Chem. Int. Edit. 40:4040-4041.

Suitable compounds are disclosed in WO03/080566, WO02/074981, WO2007/146483, WO2007136990, WO2007/103905, W02007/150011, US2007/0299086, US2007/0249605 and US2007/0213335. Other suitable compounds include inhibitors of HIF hydroxylase. HIF hydroxylase inhibitors are disclosed in United States Patent Application Publication Nos: 20070042937, 20060276477, 20060270699, 20060258702, 20060258660, 20060251638, 20060183695, 20060178317 and 20060178316 and in International Patent Application Publication Nos: WO2007/070359, WO2008/002576 and WO 2007/103905.

Other suitable compounds include compounds of formula (I): 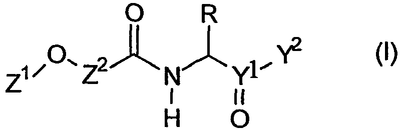 wherein
- Y² is selected from -OR' and -NR'R" wherein R' is hydrogen, or unsubstituted C₁₋₄ alkyl and R" is hydrogen, hydroxy or unsubstituted C₁₋₄ alkyl;
- Y¹ is selected from -C-, -S- and -S(O)-;
- Z² is selected from -C(O)- and -NR"- wherein R" is selected from hydrogen, hydroxy or unsubstituted C₁₋₄ alkyl;
- Z¹ is selected from hydrogen and unsubstituted C₁₋₄ alkyl; and
- R is a side chain of a naturally occurring amino acid.

Preferably Y¹ is -C- and Y² is -OH or -NH₂. Most preferably Y¹ is -C- and Y² is -OH.

Preferably Z² is -C(O)- or NR"- wherein R" is hydrogen, methyl or ethyl. More preferably Z² is -C(O)- or -NH-. Preferably Z¹ is hydrogen, methyl or ethyl, more preferably hydrogen. Most preferably Z² is -C(O)- and Z¹ is hydrogen, methyl or ethyl.

Preferably R is a side chain of alanine, valine, leucine or phenylalanine. Preferably R is a side chain of valine, leucine or phenylalanine. More preferably R is a side chain of phenylalanine, i.e. -CH₂Ph.

L-stereoisomers or D-stereoisomers of these compounds may be used.

An exemplary synthetic scheme used to obtain test compounds of formula (I) is shown below in Scheme 1. Here an amino acid is reacted with an oxalyl chloride in order to produce a compound of formula (I). In this scheme the amino acid used is phenylalanine, although it will be apparent that the same general reaction will occur with other amino acids. The first reaction yields a protected compound of the invention (the dimethyl ester form). The diacid form is easily generated through reaction with aqueous sodium hydroxide.

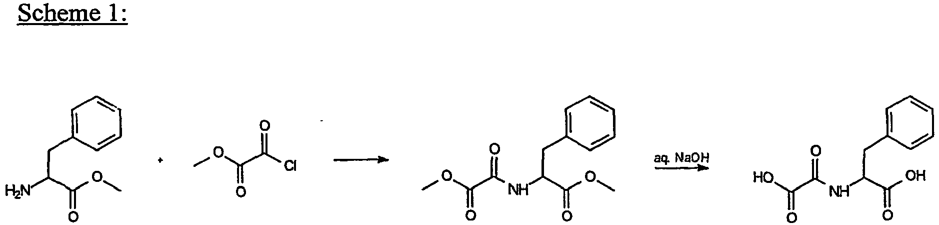

Compounds in which X is -O- or -S- or Z is other than -CO-CO-OH may by synthesised as described in Mole et al. (2003) Bioorg. Med. Chem. Lett. 13, 2677-2680 and Cunliffe et al. J. Med. Chem. (1992) 35 2652-2658.

Krebs cycle intermediates such as succinate and fumarate act as inhibitors of FTO demethylase activity. Therefore analogues of succinate and fumarate may be used to inhibit FTO activity.

In particular, the inventors have shown that the following compounds are inhibitory of Jmjd6 lysyl hydroxylase activity: an N-oxalyl amino acid such as N-oxalylglycine (NOG) or a derivative thereof, a glycine or alanine derivative, a 2-oxoacid analogue, a flavonoid or flavonoid derivative such as genistein, pyridine-2,4-dicarboxylic acid, fumarate, succinate, FG0041, FG2216 or LBE-6.

Enhancers of Jmjd6 activity include chelating agents such as pyridine-2,5-dicarboxylic acid and pyridine-2,6-dicarboxylic acid.

An inhibitor or activator of 2-OG oxygenase activity may be used to modulate lysyl hydroxylation of splicing regulatory protein or a fragment of a splicing regulatory protein comprising a lysine residue by Jmjd6. An inhibitor or activator of 2-OG oxygenase activity may be used to modulate RNA splicing.

A compound, substance or agent which is found to have the ability to affect the oxygenase (lysyl hydroxylase) activity of Jmjd6 has therapeutic and other potential in a number of contexts, as discussed. In particular modulators of Jmjd6 activity may be used in the treatment or prevention of diseases associated with defects in RNA splicing, such as genetic disorders and cancers.

A modulator of Jmjd6 lysyl hydroxylase activity may be used in a method of treating a genetic disorder. Also described is a method of treating a genetic disorder, which comprises administering to a subject in need thereof a therapeutically effective amount of a modulator of Jmjd6 lysyl hydroxylase activity. Alterations in RNA splicing can cause disease directly, modify the severity of the disease phenotype or be linked with disease susceptibility. Modulating RNA splicing by inhibiting or enhancing Jmjd6 activity to regulate RNA splicing offers a novel mechanism for treating such diseases. The treatment may prevent disease onset, reduce the symptoms of the disease or reduce susceptibility of the subject to the disease. Genetic disorders that may be treated be administering a modulator of Jmjd6 lysyl hydroxylase activity include, for example, spinal muscular atrophy, retinitis pigmosa, Prader-Willi Syndrome, Huntington disease, spinocerebellar ataxias, oculopharangyl muscular dystrophy, myotonic dystrophy, insulin resistance, cystic fibrosis, familial dysautonomia, systemic lupus erythematosus, bipolar disorder, schizophrenia, myocardial infarction, type 1 diabetes, cardiac hypertrophy, asthma, tauopathies, multiple sclerosis and elevated cholesterol. All of these diseases are associated with defective RNA splicing.

A modulator of Jmjd6 lysyl hydroxylase activity may be used in a method of treating cancer. Also described is a method of treating a cancer, which comprises administering to a subject in need thereof a therapeutically effective amount of a modulator of Jmjd6 lysyl hydroxylase activity. Splicing abnormalities are a common characteristic of cancer. In particular, it has been shown that changes in splicing regulatory protein expression can have causative roles in neoplasia. Upregulation or changes in splicing regulatory protein phosphorylation have been demonstrated in a number of cancers and a splicing regulatory protein is mutated in some colon and breast cancers. Furthermore, the inventors have shown that regulation of Jmjd6 is likely to be important for control of cell cycle progression, the abberation of which predisposes individuals to the development of cancer. Therefore, administration of a therapeutically effective amount of a modulator of Jmjd6 activity may be used to reduce tumor cell growth or metastasis and, in subjects with a genetic disposition to cancer, to prevent cancer initiation. Examples of cancers that may be treated by modulating (inhibiting or enhancing) Jmjd6 activity include, but are not limited to, breast cancer, colon cancer, myeloma and hepatoma.

The modulator of Jmjd6 lysyl hydroxylase activity, may be a known inhibitor of a 2OG-dependent oxygenase, such as an N-oxalyl amino acid such as N-oxalylglycine (NOG) or a derivative thereof, a glycine or alanine derivative, a 2-oxoacid analogue, a flavonoid or flavonoid derivative such as genistein, pyridine-2,4-dicarboxylic acid, fumarate, succinate, FG0041, FG2216 or LBE-6. The inhibitor may be a selective inhibitor of Jmjd6 activity compared to other 2-OG oxygenases.

An agent identified using one or more primary screens (e.g. in a cell-free system) as having ability to modulate oxygenase activity may be assessed further using one or more secondary screens.

Generally, an agent, compound or substance which is a modulator is provided in an isolated and/or purified form, i.e. substantially pure. This may include being in a composition where it represents at least about 90% active ingredient, more preferably at least about 95%, more preferably at least about 98%. Any such composition may, however, include inert carrier materials or other pharmaceutically and physiologically acceptable excipients, such as those required for correct delivery, release and/or stabilisation of the active agent.

We also describe compounds obtained by assay methods of the present invention, and compositions comprising said compounds, such as pharmaceutical compositions wherein the compound is in a mixture with a pharmaceutically acceptable carrier or diluent. Examples of suitable carriers or diluents are given in, for example, "Harrison's Principles of Internal Medicine". The carrier may be liquid, e.g. saline, ethanol, glycerol and mixtures thereof, or solid, e.g. in the form of a tablet, or in a semi-solid form such as a gel formulated as a depot formulation or in a transdermally administrable vehicle, such as a transdermal patch.

We also describe a method of treatment which includes administering to a patient an agent which modulates Jmjd6 oxygenase activity. Such agents may include inhibitors or activators of Jmjd6 oxygenase activity.

The therapeutic/prophylactic purpose may be related to the treatment of a condition associated with reduced or suboptimal or increased Jmjd6 levels or activity, or conditions in which have normal Jmjd6 levels, but where a modulation in activity such as an increase or decrease in Jmjd6 oxygenase activity is desirable. For example, Jmjd6 activity may be modulated in the treatment of disorders associated with abnormal RNA splicing.

A therapeutically effective amount of an agent is typically administered to a subject in need thereof. A therapeutically effective amount is an amount which ameliorates the symptoms of the condition or lessens the suffering caused to the subject by the condition.

We also describe a pharmaceutical composition, medicament, drug or other composition for such a purpose, the composition comprising one or more agents, compounds or substances as described herein, including inhibitors or activators of Jmjd6 oxygenase activity, the use of such a composition in a method of medical treatment, a method comprising administration of such a composition to a patient, e.g. for treatment (which may include preventative treatment) of a medical condition as described above, use of such an agent compound or substance in the manufacture of a composition, medicament or drug for administration for any such purpose, e.g. for treatment of a condition as described herein, and a method of making a pharmaceutical composition comprising admixing such an agent, compound or substance with a pharmaceutically acceptable excipient, vehicle or carrier, and optionally other ingredients.

The method for providing a pharmaceutical composition may typically comprise:
(a) identifying an agent by an assay method of the invention; and
(b) formulating the agent thus identified with a pharmaceutically acceptable excipient.

The pharmaceutical compositions described herein may comprise an agent, polypeptide, polynucleotide, vector or antibody described herein and a pharmaceutically acceptable excipient.

Whatever the agent used in a method of medical treatment described herein, administration is preferably in a "prophylactically effective amount" or a "therapeutically effective amount" (as the case may be, although prophylaxis may be considered therapy), this being sufficient to show benefit to the individual. The actual amount administered, and rate and time-course of administration, will depend on the nature and severity of what is being treated. Prescription of treatment, e.g. decisions on dosage etc, is within the responsibility of general practitioners and other medical doctors.

An agent or composition may be administered alone or in combination with other treatments, either simultaneously or sequentially dependent upon the condition to be treated, e.g. as described above.

Pharmaceutical compositions may include, in addition to active ingredient, a pharmaceutically acceptable excipient, carrier, buffer, stabiliser or other materials well known to those skilled in the art. In particular they may include a pharmaceutically acceptable excipient. Such materials should be non-toxic and should not interfere with the efficacy of the active ingredient. The precise nature of the carrier or other material will depend on the route of administration, which may be oral, or by injection, e.g. cutaneous, subcutaneous or intravenous.

Pharmaceutical compositions for oral administration may be in tablet, capsule, powder or liquid form. A tablet may include a solid carrier such as gelatin or an adjuvant. Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

Liposomes, particularly cationic liposomes, may be used in carrier formulations. Examples of techniques and protocols mentioned above can be found in Remington's Pharmaceutical Sciences, 16th edition, Osol, A. (ed), 1980.

The substance or composition may be administered in a localised manner to a particular site or may be delivered in a manner in which it targets particular cells or tissues, for example using intra-arterial stent based delivery.

Targeting therapies may be used to deliver the active substance more specifically to certain types of cell, by the use of targeting systems such as antibody or cell specific ligands. Targeting may be desirable for a variety of reasons, for example if the agent is unacceptably toxic, or if it would otherwise require too high a dosage, or if it would not otherwise be able to enter the target cells.

The following Examples illustrate the invention.

### Examples

### Example 1: Experimental Procedures

### Cloning, Expression and Purification of human Jmjd6 (residues 1-343)

The gene encoding human Jmjd6 (residues 1-343) was cloned into a pET-28a(+) vector (Novagen) and the protein was expressed in *E*. *coli* BL21-rosetta cells in 2TY growth medium supplemented with kanamycin (25 µgmL⁻¹) and chloramphenicol (30 µgmL⁻¹). Cells were grown at 37°C until an OD₆₀₀ of 0.6 when the temperature was dropped to 25°C and the cells induced with IPTG (0.5 mM). Cells were then harvested 16-18 hours post-induction and lysed by sonication in Tris (50 mM, pH 7.0), NaCl (300 mM), TCEP (0.2 mM) and glycerol (10%) (lysis buffer). Purification of the N-terminally His₆-tagged (N-terminus: MGSSHHHHHHSSGLVPRGSH) protein was carried out using nickel-affinity chromatography where Jmjd6 was eluted off the column using a gradient from 0 to 100% imidazole (500 mM). The protein was then analysed by SDS-PAGE and fractions containing protein of the correct molecular weight and the best purity were pooled and concentrated using a 10,000 MWCO filter (Millipore). The protein was buffer-exchanged back into lysis buffer and concentrated to 10-20 mgmL⁻¹ which was measured using a NanoDrop^{®}. Further purification of Jmjd6 (>95% as determined by SDS-PAGE analysis) was performed using size-exclusion chromatography (SEC), using a Superdex-75^{™} (300 mL, GE Healthcare) preparative grade column.

The H187A and H187A-D189A Jmjd6 variants were created from the wild-type construct (pET-28a(+)-Jmjd6 (residues 1-343)) using the QuikChange^{®} II Site-directed mutagenesis kit (Stratagene). The integrity of the mutations was confirmed by DNA sequencing (Geneservice, Oxford).

### Tandem affinity purification (TAP) and mass spectrometry (MudPIT)

A TAP-tagged Jmjd6 fusion protein was also obtained by tandem affinity purification with Jmjd6 in HEK293T-cells. The tag represents a fusion of calmodulin binding peptide (CBP), a tobacco etch virus (TEV) cleavage site and protein A. For the tandem affinity purification (TAP), full-length Jmjd6 was cloned into pECFP-N1-TAPc (Benzinger et al (2005) Molecular and Cellular Proteomics 4:785).

HEK293T cells were transiently transfected with pECFP-N1-TAPc:Jmjd6. Extracts of 2 x 10⁸ cells were prepared on ice for 15 min in lysis buffer (10 mM 2-amino-2-hydroxymethyl-propane-1,3-diol (Tris), pH 8.0, 150 mM NaCl, 1% NP-40, 5 mM dithiothreitol (DTT) supplemented with protease (pefabloc (Boehringer), pepstatin A, aprotinin, leupeptin) and phosphatase inhibitors (100 nM okadaic acid and cocktail 1 + 2 (Sigma)). The Jmjd6-CBP fusion protein was purified by affinity chromatography on an immunoglobulin column. After removal from the immunoglobulin column using TEV protease, the fusion protein was further purified by affinity chromatography on a calmodulin column. The purified protein was visualised by SDS-PAGE as shown in Figure 1.

### GFP-pulldown

HEK293T cells were transiently transfected as described below. Extracts from 1 x 10⁷ cells were prepared in 100 µl lysis buffer (20mM Tris/HCl pH 8.0, 150mM NaCl, 0.5% NP40 supplemented with protease (Pefabloc (Boehringer), pepstatin A, Aprotinin, Leupeptin) and phosphatase inhibitors (100 nM okadaic acid and cocktail 1 + 2 (Sigma)). After centrifugation supernatants were diluted to 200 µl with lysis buffer without NP40, incubated with 50 µl of GFP-nanotrap (Rothbauer et al. (2008) Molecular and Cellular Proteomics 7:282) for 1 hour at 4°C with constant mixing. After centrifugation the supernatant was removed, the beads washed twice with 1 ml of dilution buffer containing 300 mM NaCl and the proteins were eluted in SDS-sample buffer and subjected to SDS-PAGE/Western blotting.

### Co-immunoprecipitation

Monoclonal anti-U2AF65 antibody was used for immunoprecipitation experiments. Control experiments were performed without adding anti-U2AF65 antibody. Extracts of 2 x 10⁷ HeLa cells (untransfected or 24 hours after transfection with pcDNA3:Jmjd6) were prepared as described for GFP-pulldown and incubated with or without (control) anti-U2AF65 antibody for 1 hour under agitation at 4°C. Protein-G Sepharose 4 Fast Flow (Amersham Biosciences, NJ, USA) (100 µl) was added for another hour. After 3 wash steps with 20 mM Tris/HCl, pH 8.0, 150 mM NaCl, the beads were re-suspended in SDS sample buffer and subjected to SDS-PAGE/Western blotting.

### Cell culture, transfection and immunostaining

HeLa cells and HEK293T cells were cultured in Dulbecco's modified Eagle's medium (DMEM) supplemented with 10% fetal calf serum, penicillin (100 U/ml) and streptomycin (100 µg/ml) at 37°C, 5% CO₂. For microscopy cells were grown to 50-70% confluence on 18x18 glass coverslips and transfected with the indicated expression constructs using jetPEI (Polyplus transfection, Illkirch, France) or Nanofectin (PAA, Pasching, Austria) according to the manufacturer's instructions. 24 hours after transfection cells were fixed with 4% paraformaldehyde in PBS for 15 min at room temperature, permeabilised with methanol for 2 min and 1 % Triton X-100 in PBS for 10 min, stained with antibodies, counterstained with To-Pro3 (Invitrogen, CA, USA) and mounted in Vectashield (Vector Laboratories, CA, USA) As primary antibodies we used monoclonal anti-U2AF65 (SIGMA (U4758), Saint Louis, USA), monoclonal anti-SC35 (ab11826), polyclonal anti-H3K4 trimethylated (ab8580), polyclonal anti-Jmjd6 (ab10526), monoclonal anti-Y12 (ab3138) (Abcam, Cambridge, UK), monoclonal anti-GFP (Roche Applied Science, Mannheim, Germany), anti-CROP (a gift from Kazumitsu Ueda, Kyoto University, Kyoto, Japan).

### Confocal microscopy

Optical serial sections were taken with a Leica TCP SP confocal laser-scanning microscope (Leica Microsystems, Heidelberg, Germany) equipped with an oil immersion PlanApochromat 100/1.4 NA lens. Fluorochromes were visualized with an excitation wavelength of 488 nm and emission filters of 520-540 nm for GFP, and with an excitation wavelength of 633 nm and emission filter of 660-760 nm for To-Pro 3. For Cy3 excitation wavelength was 561 nm, and an emission filter of 570-580 nm was used. Image resolution was 512 x 512 pixel with a pixel size ranging from 195 to 49 nm depending on the selected zoom factor. The axial distance between optical sections was 300 nm. Each section image was averaged from four successive scans. The 8-bit gray scale single-channel images were overlaid to an RGB image assigning a false colour to each channel, and then assembled into tables using Adobe Photoshop 8.0 and ImageJ 1.32j software.

### RNase A treatment

After washing with PBS HeLa cells were fixed in methanol for 2 min at 25°C and treated with RNase A (100 mg/ml in PBS) for 2 hours at 25°C. Cells were then fixed in 4 % paraformaldehyde for 15 min and immunostained as described above.

### Assay for Jmjd6 oxygenase activity

Standard assays consisted of the substrate mixture: peptide (100 µM), 2OG (500 µM), ascorbate (100 µM) in Tris (50 mM, pH 7.5); and the enzyme mixture: FeNH₄SO₄ (100 µM) and Jmjd6 (10 µM) in Tris (50 mM, pH 7.5). The reaction was initiated by mixing the substrate and enzyme mixtures and incubating at 37°C for 30-60 minutes. The reaction was quenched by adding TFA to a final concentration of 0.1 % and incubating on ice. Hydroxylation of the peptide was analysed by a MALDI TOF micro MX mass spectrometer (Waters Micromass); where the assay mixture (1 µL), along with α-cyano-4-hydroxycinnamic acid MALDI (CHCA) matrix in 60% acetonitrile/0.1% TFA (1 µL), was spotted directly onto the target plate. To determine whether modification of peptide was Jmjd6-dependent assays were performed omitting either 2OG or Fe(II). To determine whether Jmjd6 was inhibited by N-oxaloylglycine (NOG), NOG (24mM) was added to the substrate component of the assay system.

### MS/MS analysis of hydroxylated peptide d

MS/MS analysis was carried out using a Ultraflex III MALDI TOF-TOF (Bruker Daltonics) mass spectrometer and experiments were acquired using the potential LIFT technique (Bruker Daltonics), based on post source and post-decay acceleration of fragment ions. MS/MS spectra were annotated using FlexAnalysis software and transferred to BioTools for sequence evaluation. The Sequence Editor tool was used to define the peptide sequence variants to be matched to the actual MS/MS spectra in BioTools. Assay mixtures (with and without Jmjd6) (1 µL) were spotted directly onto the MALDI target plate, followed by CHCA matrix (1 µL), and then allowed to dry. Mass spectra were acquired in the positive reflector mode with 17 kV acceleration voltage. The mass corresponding to peptide d and peptide d +16, equivalent to one hydroxylation, were identified and subsequent MS/MS spectra were acquired by post source decay experiments in positive reflector mode.

### NMR analysis of hydroxylated peptide d

Peptide d (residues 267-278 of Luc-like2), was modified by incubation with Jmjd6 in the presence of ascorbate, 2OG and Fe(II) overnight at 22°C. The reaction was quenched by the addition of methanol (20%). Precipitate was removed by centrifugation and the supernatant purified by HPLC using a Synergi™ Hydro-RP (100 x 21.2 mm). Peptide was eluted using a gradient of acetonitrile in 0.1% trifluoroacetic acid and then lyophilised. To prepare the peptide for NMR analysis the sample was lyophilised a second time from ²H₂O and resuspended in 6 µL of this solvent. 1D ¹H and 2D COSY NMR experiments were performed on a Bruker AVII 500 spectrometer using a 1mm microprobe.

### ¹⁸O₂ dioxygen experiment with peptide d

The hydroxylation of peptide d by Jmjd6, under standard assay conditions, was performed under an atmosphere of ¹⁸O₂. A rubber septum sealed reaction vessel, containing the substrate mixture, was evacuated and flushed with argon no less than three times before being filled with ¹⁸O₂ gas (>98 % CK gases). The reaction was initiated by injecting the enzyme mixture (Jmjd6 and Fe(II)) through the septum into the vessel. The reaction was carried out for one hour before it was quenched by freezing and then addition of TFA (0.1 %). Products were analysed by MALDI-TOF mass spectrometry.

### Substrate Specificity of Jmjd6

All peptides tested as potential substrates of Jmjd6 were synthesised in-house by a Intavis MultiPep automated multiple synthesiser, using Tentagel amide resin (Intavis). Fluorenylmethoxycarbonyl (Fmoc)-protected amino acids were coupled with N,N'-diisopropylcarbodiimide (DIC)/1-hydroxybenzotriazole (HOBt). Cleavage of the completed peptides from the resin used CF₃CO₂H (TFA)/triethylpropylsilane (95/2.5). Predicted masses were confirmed by MALDI-TOF mass spectrometry. Standard assay conditions were used and are described above.

To determine the pH profile of Jmd6 standard peptide assays were performed over a pH range of 6 to 9. Buffers used were piperazine-N,N'-bis(ethanesulfonic acid) (PIPES) (pH 6.0 and 6.5), 4-(2-hydroxyethyl)-1-piperazineethanesulfonic acid (HEPES) (pH 7.0 to 8.0) and N,N-bis(2-hydroxyethyl) glycine (BICINE) (pH 8.5 and 9.0). Inhibition assays were performed under identical conditions as described for the standard assays except either succinate (1 mM) or fumarate (1 mM) were included in the substrate mixture.

### In vivo double reporter splicing assay

HeLa cells were transiently transfected with relevant plasmids using Lipofectamin 2000 (Invitrogen, CA, USA) according to the manufacturer's instructions. 24 hours after transfection cells were harvested and luciferase activitiy was measured using the Dual-Luciferase Reporter Assay System (Promega, Madison, USA). β-Galactosidase activity was measured with the chemiluminescent reporter gene assay system Galacto-Light (Applied Biosystems, CA USA).

### Effect of RNAi-mediated depletion of JMJD6 on α-tropomyosin alternative splicing.

6URE α-TM minigene has been described elsewhere (Gromak and Smith (2002), Nucleic Acids Research 30(16): 3548-3557). Transient transfections in HeLa cells and RT-PCR RNA analysis using radioactively labelled primers has been performed as described in Gromak et al. (2008), RNA 14(2): 359-366. RNAi procedure was carried out as described in Wagner and Garcia-Blanco (2002), Molecular Cell 10(4): 943-949.

RT-PCR primers for detection of actin mRNA were 5'-CGTGATGGTGGGCATGGGTCAG-3' and 5'-CTTAATGTCACGCACGATTTCC-3' and for PSR mRNA- 5' GGCACAACTACTACGAGAGC-3' and 5'-TTTGGCACCTTGTAGTCTTCC-3'.

Western blotting was performed by standard immunoblotting procedure with ECF western blotting kit (Amersham Biosciences) on 25-50 µg of total HeLa cell protein extract. Antibodies for western blot analysis were rabbit polyclonal anti-JMJD6 antibody (Abcam), rabbit polyclonal anti-actin antibody (Sigma) and anti-rabbit fluorescein-conjugated antibody (Sigma).

### Example 2: Identification of Jmid6 Interacting Proteins

To investigate the function of Jmjd6 in cells we assayed for proteins that interact with it using the tandem-affinity purification procedure and HEK293T cells with mass spectrometry (MS) based identification. After excluding non-selective interactions these analyses revealed that a significant proportion (20/37) of the remaining potential Jmjd6 interacting proteins were involved in RNA metabolism, processing and splicing (Table 1).

**Table 1: Splicing regulatory Proteins that Interact with Jmjd6**

| | | |
|---|---|---|
| ATP-dependent RNA helicase DDX41 | Q9UJV9 | RNA metabolism/splicing |
| CROP protein | Q95232 | RNA metabolism/splicing |
| Poly-U binding splicing factor PUF60 | Q9UJY7 | RNA metabolism/splicing |
| Putative RNA-binding protein Luc7-like 1 | Q9NQ29 | RNA metabolism/splicing |
| Putative RNA-binding protein Luc7-like 2 | Q9Y383 | RNA metabolism/splicing |
| Splicing factor arginine/serine rich 11 (p54) | Q05519 | RNA metabolism/splicing |
| Arginine/serine-rich coiled coil protein 1 | Q96127 | RNA metabolism/splicing |
| Probable ATP-dependent RNA helicase DDX46 | Q7L014 | RNA metabolism/splicing |
| RNA binding motif protein 25 | P49756 | RNA metabolism/splicing |
| Acinus (Apoptotic chromatin condensation inducer in the nucleus) | Q9UKV3 | RNA metabolism/splicing |
| Probable ATP-dependent RNA helicase DDX17 (p72) | Q92841 | RNA metabolism/splicing |
| RNA binding protein | Q9UQ39 | RNA metabolism/splicing |
| Pre-mRNA-processing factor 40 homolog A | Q75400 | RNA metabolism/splicing |
| Splicing factor U2AF 35kD subunit | Q01081 | RNA metabolism/splicing |
| Splicing factor U2AF 65kD subunit | P26358 | RNA metabolism/splicing |
| RNA-binding protein 39 (Splicing factor HCC1) | Q14498 | RNA metabolism/splicing |
| RNA polymerase-associated protein RTF1 homolog | Q92541 | RNA metabolism/pre-mRNA processing |
| Cleavage and polyadenylation specificity factor 6 | Q16630 | RNA metabolism/pre-mRNA processing |
| | | |
| Nucleolar RNA helicase II (DDX21) | Q9NR30 | RNA metabolism/Nucleolus |
| Nucleolar phosphoprotein p130 | Q14978 | RNA metabolism (RNA Pol 1 + CK2 associated)/Nucleolus |
| Treacle protein (Treacher Collins syndrome protein) | Q13428 | May be involved in nucleolar-cytoplasmic transport |
| H/ACA ribonuleoprotein complex subunit 4 (Dyskerin) | 060832 | ribosome biogenesis |
| | | |
| Nuclease sensitive element binding protein 1 | P67809 | DNA binding |
| DNA-binding protein A | P16989 | DNA binding |
| Parafibromin (Cell division cycle protein 73 homolog) | Q6P109 | Interacts with the RNA polymerase II large subunit (RPB 1) and LEO1. Interacts with a Set1-like complex that has histone methyltransferase activity and methylates histone H3. |
| Bromodomain-containing protein 4 | 060885 | DNA binding |
| | | |
| Eukaryotic translation initiation factor 3 subunit 4 | 075821 | translation/ribosome |
| Eukaryotic translation initiation factor 3 subunit 8 | Q99613 | translation/ribosome |
| Elongation factor 1-alpha 2 | Q05639 | translation/ribosome |
| | | |
| NF-kappa-B-activating protein | Q8N5F7 | Regulator of TNF and IL-1 induced NF-kappa-B-activation |
| | | |
| Phosphatidylinositol-4 phosphate 5-kinase type II alpha | P48426 | |
| Casein kinase 2A1 | P68400 | |
| Phosphatidylinositol-4 phosphate 5-kinase type II gamma | Q8TBX8 | |
| Phophatidylinositol-4 phosphate 5-kinase type II beta | P78356 | |
| | | |
| Hypothetical protein FLJ32377 | Q96MH4 | unknown |
| RNF187 protein | Q6PJR0 | unknown |
| Small acidic protein | O00193 | unknown |
| Multiple myeloma tumor-associated protein 2 | Q9BU76 | unknown |
| Hepatoma derived growth factor 2 | Q7Z4V5 | unknown |

We then tested for the interaction of Jmjd6 with the splicing factor U2 snRNP auxiliary factor 65 KDa subunit (U2AF65) and cisplatin resistance-associated overexpressed protein (CROP) using GFP-pulldown assays. Lysates from HEK293 cells expressing Jmjd6-GFP or control HEK293 cells expressing GFP were subjected to GFP-pull down experiments. The results demonstrated an interaction of ectopically expressed GFP-Jmjd6 with endogenous CROP and U2AF65 in HEK293T cells (Figure 3).

A plasmid expressing a YFP (yellow fluorescent protein)-U2AF65 fusion protein was then expressed in HEK293T cells together with untagged Jmjd6. Imunoprecipitation analyses demonstrated an interaction between Jmjd6 and U2AF65 (Figure 3).

An interaction between endogenous U2AF65 and Jmjd6 was observed by co-immunoprecipitaton with anti-U2AF65 from a HeLa cell extract (Figure 2).

### Example 3: Analysis of Sub-cellular Localisation of Jmjd6

We then analysed the sub-cellular localisation of Jmjd6 using HeLa cells and an anti-Jmjd6 antibody. Endogenous Jmjd6 was found to localise in the nucleoplasm in a diffuse fashion and to overlap with nuclear speckles/interchromatin granules (dynamic structures enriched in RNA splicing factors located in interchromatin regions of the nucleoplasm) in areas excluding the nucleolus (Figure 4).

Co-localisation with the non-snRNP spliceosome component SC-35 in speckled areas was observed, suggesting association with interchromatin granules and perichromatin fibrils. Partial colocalisation of Jmjd6 in speckled areas was observed with U2AF65 (Figure 5), which occurs in spliceosomes, supporting the MS-based interaction assignments. In contrast Jmjd6 was not observed in heterochromatin regions. A similar distribution pattern was observed for analyses with the GFP-Jmjd6 fusion.

Co-staining analyses with an antibody against histone H3K4 (trimethylated), which is indicative of euchromatin, suggested that Jmjd6 is not present in euchromatin regions (Figure 4).

Staining of HeLa cells with anti-Jmjd6 antibody ab10526 in all phases of the cell cycle showed that Jmjd6 was not associated with chromosomes (Figure 6). The Jmjd6 speckles broke up in pro- or early metaphase and during metaphase Jmjd6 was evenly distributed in the cytoplasm. In anaphase stronger staining was observed in the space between the separating chromatids. In telophase Jmjd6 was again seen in speckles in the nucleus. This redistribution of Jmjd6 during mitosis is similar to the behaviour of snRNPs and SC-35 in mitotic cells (Spector and Maniatis (1991), Embo J. 10(11): 3467-81).

Upon treatment of methanol fixed HeLa cells with RNAse (Figure 7) the Jmjd6 staining pattern changed; the speckles were no longer observed, with only weak nucleoplasmic signals remaining. This observation is similar to the response of small-nuclear riboproteins to RNAse treatment.

### Example 4: Analysis of Jmjd6-Bindine Partners

Bioinformatic sequence analyses revealed that most of the proteins identified as binding partners of Jmjd6 were rich in arginine and serine residues, and many possessed Arg-Ser (RS) domains. The RS domain predominantly consists of an imperfect 8-amino acid repeat motif (consensus sequence, Ser-Arg-Ser-Arg-Asp/Glu-Arg-Arg-Arg) and has been implicated in the mediation of protein-protein interactions, spliceosome assembly (Umehara et al. (2003), Biochemical and Biophysical Research Communications 301(2): 324-329) and RNA association (Nikolakakiet al. (2008), Biochimica et Biophysica Acta, General Subjects 1780(2): 214-225).

To determine whether U2AF65, CROP and LUC7-like2 were substrates of Jmjd6, multiple peptides were synthesized based on the RS domains of these proteins (Figure 8). Initial peptides were synthesized with dimethylated arginine residues because it was speculated that Jmjd6 may be an arginine demethylase (Figure 9). To determine whether any of these peptides were Jmjd6 substrates, incubations were carried out in the presence of oxygen, ascorbate, Fe(II) and 20G; and peptide modifications were analyzed by MALDI-TOF MS.

In the case of peptides a, b and c, a +16 mass shift relative to un-reacted peptide was observed (Figure 10); this is consistent with the incorporation of one oxygen atom into the peptide. The +16 mass shift in peptide d was shown to be 20G- and Fe(II)- dependent, and completely inhibited by an established 20G- and Fe(II)- dependent oxygenase inhibitor, N-oxalylglycine (Hewitson et al. (2002) J. Biol. Chem. 277(29): 26351-26355) (Figure 11) as well as the citric acid cycle intermediates succinate and fumarate. There was no evidence for arginine demethylation with these peptides, i.e. if occurring it was below the limits of our detection at about 5% substrate to product conversion.

Jmjd6 active site variants in which one or two of the proposed Fe(II) binding residues (Wolf et al (2007) EMBO Reports 8:465) were mutated (H187A and H187A-D189A) are inactive, implying an intact Fe(II) binding centre is required for catalysis.

### Example 5: Identification of Site and Stereochemistry of Hydroxylation by Jmjd6

Evidence for the position of hydroxylation came from MS/MS analyses. Thus, for peptide d, the 'b' and 'y' series of fragmented ions, derived from cleavage of the peptide bond between the α-amino group nitrogen and carboxylic acid carbon, respectively, are consistent with the hydroxylation of lysine-3 in peptide d, corresponding to lysine 269 in LUC-like2 (Figure 12).

Further evidence supporting hydroxylation of lysine3 came from N-terminal sequence analysis of the product peptide d by Edman degradation, using DL and DL allo-hydroxylysine as a reference.

NMR analyses on the hydroxylated product from peptide d identified the site of hydroxylation as C-5 on lysine-269, the same position as observed for lysyl-hydroxylaton in collagen (Yamauchi et al (1982), PNAS USA 79(24): 7684-8) (Figure 13).

Significantly, Jmjd6 was able to hydroxylate two lysine residues on peptide e, as seen by a +16 and a +32 mass shift in peptide by MALDI TOF. When either lysine in peptide d was individually mutated to an arginine only a single hydroxylation was observed. Mutation of both of these lysines to arginines resulted in no hydroxylation by Jmjd6 (Figure 15).

We did not observe Jmjd6 catalysed arginine demethylation on the SR peptides that we studied. We demonstrated that lysyl hydroxylation occurs on histone peptides, as seen in SR proteins (Figure 16). As with the SR peptides with our histone peptides we did not observe arginine demethylation.

Overall the results reveal that lysyl hydroxylation is the dominant catalytic activity of Jmjd6, with arginine demethylation occurring at a much lower rate, if at all, at least with our assay conditions.

Peptides f and g, which do not contain any lysine residues, were not hydroxylated by Jmjd6.

**Table 2: Hydroxylation of Histone peptides**

| No | Peptide sequence | Length of peptide | Protein sequence based on | activity |
|---|---|---|---|---|
| 1 | SGR(me2sym)GKGGKGLGKGGAK | 16mer | Histone H4 | +16 no demethylation |
| 2 | SGR(me2asym)GKGGKGLGKGGAK | 16mer | Histone H4 | +16 no demethylation |
| 3 | SGRGKGGKGLGKGGA | 15mer | Histone H4 | +16 |
| 4 | KGGKGLGKGGAKRHR | 15mer | Histone H4 | +16 |
| 5 | AR(me2sym)TKQTELRKSTGGKAPPK | 18mer | Histone H3 | Double hydroxylation no demethylation |
| 6 | AR(me2asym)TKQTARKSTGGKAPPK | 18mer | Histone H3 | Double hydroxylation no demethylation |
| 7 | ARTKQTARK(me3)STGGKA | 15mer | Histone H3 | no |
| 8 | ARTKQTARK(me2)STGGKA | 15mer | Histone H3 | no |
| 9 | ARTKQTARK(me1)STGGKA | 15mer | Histone H3 | no |
| 10 | ARTKQTARKSTGGKA | 15mer | Histone H3 | yes |
| 11 | ARTKQTARK(acetyl)STGGK(acetyl)A | 15mer | Histone H3 | no |
| 12 | ARTK(me3)QTARK(acetyl)STGGK(acetyl)A | 15mer | Histone H3 | no |
| 13 | QLATKAARKSAPATG | 15mer | Histone H3 | no |
| 14 | SGRGKQGGKARAKTR | 15mer | Histone H2a | +16 |
| 15 | SGR(me2asym)GKQGGKARAKTR | 15mer | Histone H2a | +16 |
| 16 | APAPKKGSKKAVTKA | 15mer | Histone H2b | +16 |
| 17 | GSKKAVTKAQKKDSK | 15mer | Histone H2b | +16 |

To investigate the stereochemistry of hydroxylation by Jmjd6, a hydroxylated Luc7-like peptide sequence (NP*K*RSRSREHRR) was synthesized by inserting 2S, 5R hydroxyl lysine at the postion of hydroxylation. The synthetic and enzymatically prepared (hydroxylation of Jmjd6) products were the compared and shown to be the same by NMR.

### Synthetic preparation of NPK(OH)RSRSREHRR

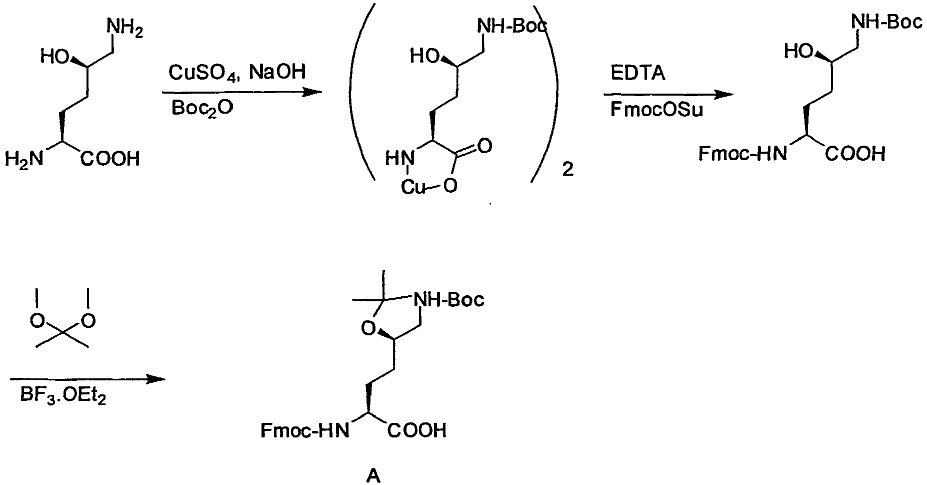

Compound A (Fmoc-2S,5R-hydroxyl lysine (Boc-oxazolidine)) was synthesized as described by Spetzler et al. (Spetzler, J.C.H.-J.T., Masked side chain aldehyde amino acids for solid phase synthesis and ligation. Tetrahedron Letters, 2002. 43: p. 2303-2306). The peptide sequence NPK(OH)RSRSREHRR (where K(OH) = hydroxylated lysine) with a C-terminal amide (where K(OH) is 5R-hydroxyl lysine) was synthesised using a CS-Bio CS336 synthesiser using PL-AMS resin (Polymer Labs aminomethylstyrene resin, 0.4 mmol/g) and the Rink Amide Linker. Starting materials used for synthesis were standard Fmoc-protected amino acids except for lysine. Previously synthesized compound A is used in place of lysine. Activation of amino acids was carried out using HOBt (1-hydroxybenzotriazole, 0.5 M solution in DMF) and DIC (diisopropylcarbodiimide, 0.5 M solution in DMSO). In a reaction vessel having pre-swelled resin, each amino acid dissolved in 1 mL DMF, was added. Immediately DIC (2 mL) and HOBt (2 mL) solutions were added and the activation reaction was allowed to proceed for 30 min. Each amino acid was allowed to react with the resin-bound peptide for 2 h, with continuous shaking, after which resin was washed with DMF. After washing, the Fmoc group was removed with 20 % piperidine in DMF/DMSO (DMF:DMSO ratio 3:1). Then, the next Fmoc-protected activated amino acid was added, and the cycle was repeated. Once the final amino acid had been coupled and the Fmoc group removed, the resin was removed from the reaction vessel, washed several times with DMF, and then with DCM. It was dried overnight in a vacuum desiccator. The peptide was cleaved from the resin and the side-chain protecting groups (Boc and Boc and an oxazolidine ring in the case of hydroxyl lysine derivative) were removed using a CF₃COOH-based cleavage cocktail; the resin was allowed to stand in 5 mL of this cleavage cocktail, without stirring, for 3 hours. The CF₃COOH solution, now containing the free peptide, was filtered off, and concentrated to 0.5 mL by bubbling N₂ gas through it The peptide was then precipitated from solution using ice-cold diethyl ether, centrifuged and solid peptide was lyophilized in 0.1 % aq. CF₃COOH.

### Hydroxylation of NPKRSRSREHRR by Jmjd-6

Standard assays consisted of the substrate mixture and the enzyme mixture as follows:

| **Enzyme Mix** | **Substrate Mix** |
|---|---|
| 50µL FL-JMJD6 | 20µL peptide (10mM) (in ammonium acetate) |
| 20µL Fe(II) (40 µM) | 20µL 2-OG (50mM) |
| 30µL ammonium acetate (10mM) | 60µL ammonium acetate (10mM, pH 7.5) |

The reaction was initiated by mixing the substrate and enzyme mixtures and incubating at 37°C. For large scale hydroxylation of peptide, it was carried out in 30 Eppendorf tubes such that total amount of peptide used was 10 mg. After two hours, hydroxylation of the peptide was analysed by a MALDI TOF micro MX mass spectrometer (Waters Micromass); where the assay mixture (1 µL), along with α-cyano-4-hydroxycinnamic acid MALDI (CHCA) matrix in 60% acetonitrile/0.1% TFA (1 µL), was spotted directly onto the target plate. Hydroxylation was further carried out by leaving the assay mixture overnight at room temperature till peptide showed complete hydroxylation.

### Purification and Analysis of NPK(OH)RSRSREHRR Prepared by Synthesis and Jmjd6 catalysis

HPLC purification of peptides was carried out using a Vydac C18 Peptide column (5-10 µM particle size, 22 mm diameter, 200 mm length) using Waters Quattro micro system. Peptides were purified using RP-HPLC with a gradient of 0-90% for 15 minutes and 90%-70% for next 15 minutes using water to acetonitrile, with 0.1 % CF₃COOH.

MALDI analyses on the peptides was carried out using a Ultraflex III MALDI TOF-TOF (Bruker Daltonics) mass spectrometer. Peptide samples dissolved in milliQwater (1 µL) were spotted directly onto the MALDI target plate, followed by CHCA matrix (1 µL), and then allowed to dry. Mass spectra were acquired in the positive reflectron mode with 17 kV acceleration voltage. The desired mass was for the purified synthetic and hydroxylated peptides was observed by MALDI.

All NMR experiments were recorded on a Bucker AVIII 700 (with inverse cryoprobe optimised for ¹H observation and running TOPSPIN 2 software) and reported in ppm relative to D₂O (δ_{H} 4.72), the deuterium signal was used as an internal lock signal and the HDO signal was suppressed by presaturating its resonance. All samples prepared in D₂O were transferred to 2 mm NMR tube. The NMR tube was centrifuged for few seconds using a hand centrifuge. Data was analyzed using TOPSPIN 2.

¹H NMR, HSQC and selective 1D TOCSY NMR analysis of the synthetic and enzymatically prepared peptides demonstrated the spectra were the same within error, i.e. demonstrated the presence of a hydroxy group at the C-5 lysine position in the product of the Jmjd6 catalyzed reaction.

### Example 6: Identification of Origin of Hydroxyl Group

Incubation under an ¹⁸O₂ atmosphere revealed the origin of the Jmjd6 introduced hydroxyl group as from dioxygen gas (Figure 14).

### Example 7: Specificity of Jmjd6

To investigate the specificity of Jmjd6 towards variations in the sequence and length of peptides derived from U2AF65 and LUC7-like2 multiple peptides were synthesized and tested. The results are shown in Tables 3 and 4. These experiments demonstrated Jmjd6 activity does not require a clearly defined consensus sequence. The shortest peptide hydroxylated by Jmjd6 was 12 amino acids long. Because lysine-methylation status can regulate protein biosynthesis, we investigated whether Jmjd6 can tolerate modifications to the lysine residue that is hydroxylated. Methylation (tri, di and mono) of lysine residues inhibits hydroxylation of those residues by Jmjd6. This raises the possibility that hydroxylation of the lysine side chain methylenes can regulate One modifications, or vice versa.

**Table 3: Hydroxylation of U2AF65 peptides**

| No | Peptide sequence | Length of peptide | Protein sequence based on | activity |
|---|---|---|---|---|
| 1 | SRDRRRRSR | 9mer | U2AF65 | no |
| 2 | SRDRARRSR | 9mer | U2AF65 | no |
| 3 | SRDRK(Me₃)RRSR | 9mer | U2AF65 | no |
| 4 | SRDRRRRSR | 9mer | U2AF65 | no |
| 5 | RDRKRRS | 7mer | U2AF65 | no |
| 6 | DRKRR | 5mer | U2AF65 | no |
| 7 | RKR | 3mer | U2AF65 | no |
| 8 | KR | 2mer | U2AF65 | no |
| 9 | SHSRSRSR(Me_{2sym})DRKRRSRS (peptide a) | 16mer | U2AF65 | yes |
| 10 | SHSRSR(Me_{2sym})SRDRKRRSRS (peptide b) | 16mer | U2AF65 | yes |
| 11 | SHSRSRSRDRKRRSRS | 16mer | U2AF65 | yes |
| 12 | HSRSRSRDRKRRSRS | 15mer | U2AF65 | no |
| 13 | SRSRSRDRKRRSRS | 14mer | U2AF65 | no |
| 14 | RSRSRDRKRRSRS | 13mer | U2AF65 | no |
| 15 | SRSRDRKRRSRS | 12mer | U2AF65 | no |
| 16 | RSRDRKRRSRS | 11mer | U2AF65 | no |
| 17 | DRKRRSRSRDRR | 12mer | U2AF65 | no |
| 18 | DRKRRSRSRDRRNR | 14mer | U2AF65 | no |
| 19 | RDKENRHRKRSHSRSRS | 17mer | U2AF65 | yes |

**Table 4: Hydroxylation of LUC7-like2 peptides**

| No | Peptide sequence | Length of peptide | Protein sequence based on | activity |
|---|---|---|---|---|
| 1 | NPKRSRSR(Me_{2sym}))EHRRHRSR (peptide c) | 16mer | LUC-like2 | yes |
| 2 | NPKRSRSR(Me_{2sym})EHRRHRSR | 16mer | LUC-like2 | yes |
| 3 | NPKRSRSR(Me₁)EHRRHRSR | 16mer | LUC-like2 | yes |
| 4 | NPKRSRSEHRRHRSR | 15mer | LUC-like2 | yes |
| 5 | NPKRSRS | 7mer | LUC-like2 | no |
| 6 | NPKRS | 5mer | LUC-like2 | no |
| 7 | NPKR | 4mer | LUC-like2 | no |
| 8 | PKR | 3mer | LUC-like2 | no |
| 9 | NPKRSRSREHRRHRS | 15mer | LUC-like2 | yes |
| 10 | NPKRSRSREHRRHR | 14mer | LUC-like2 | yes |
| 11 | NPKRSRSREHRRH | 13mer | LUC-like2 | yes |
| 12 | NPKRSRSREHRR (peptide d) | 12mer | LUC-like2 | yes |
| 13 | NPKRSRSREHR | 11mer | LUC-like2 | moderate |
| 14 | NPKRSRSREH | 10mer | LUC-like2 | no |
| 15 | NPKRSRSRE | 9mer | LUC-like2 | no |
| 16 | SHSKNPKRSRSREHRR (peptide e) | 16mer | LUC-like2 | double hydroxylation |
| 17 | SHSRNPKRSRSREHRR | 16mer | LUC-like2 | yes |
| 18 | SHSKNPRRSRSREHRR | 16mer | LUC-like2 | yes |
| 19 | SHSRNPRRSRSREHRR | 16mer | LUC-like2 | no |
| 20 | NPKKSKSREHRR | 12mer | LUC-like2 | double |
| 21 | NPKKSKSKEHRR | 12mer | LUC-like2 | double |
| 22 | NPKKSKSKEHKK | 12mer | LUC-like2 | double |
| 23 | NPKRSRSKEHKK | 12mer | LUC-like2 | yes |
| 24 | NPKRSRSREHKK | 12mer | LUC-like2 | yes |
| 25 | NPKK(Me₃)SRSREHRR | 12mer | LUC-like2 | yes |
| 26 | NPK(me3)RSRSKEHKK | 12mer | LUC-like2 | no |
| 27 | NPR(me2aym)RSRSKEHKK | 12mer | LUC-like2 | no |
| 28 | NPR(me2sym)RSRSKEHKK | 12mer | LUC-like2 | no |
| 29 | NPR(me1)RSRSKEHKK | 12mer | LUC-like2 | no |

### Example 8: Role of Jmjd6 in RNA Splicing

To investigate whether Jmjd6 can regulate RNA splicing, we employed an *in vivo* double reporter assay (Nasim et al (2002) Nuc. Acid. Res. 30: e109) comprising a β-galactosidase gene, followed by an intron and a luciferase gene under control of an SV40 promoter. Upon splicing, a β-galactosidase-luciferase fusion protein is produced leading to qualifiable activities of both enzymes. In the absence of splicing a stop codon within the intron prevents the expression of downstream luciferase and only β-galactosidase activity is observed. Over-expression of Jmjd6 inhibited constitutive splicing of the reporter by up to 70%.

To test the role of Jmjd6 in the regulation of alternative splicing *in vivo,* we employed RNAi technology to knock down the Jmjd6 protein. The Jmjd6 knock down efficiency was estimated to be around 80% at the level of mRNA and protein confirmed by the RT-PCR and Western blot analyses (see Figure 17A). To study the regulation of alternative splicing we have used the α-tropomyosin (α -TM) gene construct.

This construct recapitulates splicing regulation of the α-TM gene and contains exons 1, 3 and 4 surrounded by their intronic regulatory sequences (Figure 16B). Although the α-TM minigene lacks the mutually exclusive exon 2, this does not interfere with the splicing regulation of exon 3. Splicing of α-TM minigene construct generates two mRNA isoforms: 134 and 14. Repression of exon 3 splicing is mediated by negative regulatory elements surrounding this exon: the polypyrimidine tract (PPT), Upstream and Downstream Regulatory Elements (URE and DRE).

The amount of both α-TM spliced isoforms was determined by RT-PCR analysis using primers shown in Figure 17B in Jmjd6-knocked down and mock-treated cells, transiently transfected with α-TM minigene construct. As seen in Figure 17C, HeLa cells treated with Jmjd6 siRNA produced an increased amount of 14 spliced isoform (from average 22 to 39%), suggesting that Jmjd6 is involved in the regulation of α-TM alternative splicing.

Overall the results reveal that Jmjd6 is a lysyl hydroxylase active on RNA splicing regulatory (SR) proteins. Evidence that Jmjd6 hydroxylates RS-domains and regulates mRNA splicing *in vivo* reveals a new regulatory mechanism at the interface between oxygen and protein synthesis. SR proteins are ubiquitously involved in mRNA splicing. Hydroxylation of the lysyl 5-position may well influence regulatory modifications, including acetylation, methylation or ubiquitination at the adjacent Nε lysyl-amino group or glycosylation on hydroxylysine as in collagen.

### Example 9: Comparison of Full-Length and Truncated Jmj6 Polypeptides

Standrard assays for Jmj6 hydroxylation activity were carried out using full-length Jmjd6 (SEQ ID NO:1), a C-terminally truncated Jmjd6 polypeptide (residues 1 to 343 of SEQ ID NO: 1) and a C- and N- terminally truncated Jmjd6 polypeptide (residues 25 to 338 of SEQ ID NO: 1). Peptide d was used as a substrate. As shown in Figures 18 and 19, both the truncated Jmjd6 polypeptides and full-length Jmjd6 hydroxylated peptide d.

### Example 10: Inhibition of Jmjd6

Standard assays for Jmjd6 were carried out as described above, except 100 µM of various inhibitors were added to the substrate (peptide d) mixture to give a 10:1 ratio of inhibitor to substrate (peptide d). A control without inhibitor was included as a reference. The results are shown in Figure 20.

The most potent inhibitors of Jmjd6 activity were pyridine-2, 4-dicarboxylic acid followed by fumarate and succinate. Interestingly, the addition of pyridine-2,5-dicarboxylic acid and pyridine-2,6-dicarboxylic acid (structural isomers of pyridine-2,4-dicarboxylic acid) to the assay resulted in complete (100%) hydroxylation of peptide d, as observed by MALDI. This is most likely due to their chelating effect where inactivating metals are removed from the metal binding site of Jmjd6. FG0041, FG2216 and LBE-6-3 did have a slight (~50%) inhibitory effect on activity while NOG and N-oxalyl-D-phenylanine (NOFD) had little effect (<20%) at the concentrations tested. It should be noted that when 12 mM NOG was included in the assay mixture hydroxylation of peptide d was completely inhibited, as observed by MALDI.

### Example 11: Characterisation of important domains for homo-oligomerisation of Jmjd6: the Jmjd6 N-terminus is responsible for homo-oligomer formation

Jmjd6 forms homo-oligomers. To investigate the nature of the oligomerisation domain at first a number of deletion and point mutations was constructed. Two point mutations were designed to change the Fe(II) binding site and thus to abolish catalytic activity of the protein. Such mutants can not catalyse hydroxylation of peptide substrates *in vitro.* In an additional mutant the amino acids that show homology with AT-hook motifs were exchanged. Figure 21 shows all mutants schematically.

A fluorescence two-hybrid assay was used (Zolghadr et al., 2008). A fluorescent bait (RFP-fusion protein, Cherry) was fused to a modified lac repressor and thus, when expressed in cells carrying a chromosomal lac-operator array, is concentrated at the integration sites of the operater. Fluorescent prey proteins (GFP fusion proteins) are then co-expressed and assayed for their co-localization with the bait. In this system Jmjd6-Cherry was used as bait and co-localization with Jmjd6-GFP and its mutants was determined (see Figure 22). As shown in Figure 22A, both proteins co-localize in one single dot in the nucleus. This indicates homo-oligomerisation. N-terminal deletion mutants were then tested. Up to a deletion from 1-25 (Fig. 22B-C) both Jmjd6 fusion proteins still co-localise in one dot. However, when the first 48 amino acids are deleted, co-localisation is abolished (Figure 22D). This suggests that the oligomerisation domain of Jmjd6 lies between amino acids 25 and 48. C-terminal deletions Jmjd6Δ63-403 and Δ338-403 did not show altered oligomerisation (Figure 22E and F). Jmjd6Δ338-403 is remarkable in its distribution. Almost all of it is found in the nucleolus (see later). The active site mutants and the AT-hook mutant remained able to oligomerise (data not shown). Deletions that reached into the JmjC domain aggregated in the nucleus and could not be analysed.

### Example 12: Interaction of Jmjd6 with U2AF-65 depends on N-terminal and C-terminal sequences

The binding sites for U2AF-65 on Jmjd6 were analysed using a GFP nanotrap assay (Rothbauer et al., 2008). A fusion protein of GFP with the above mentioned Jmjd6 mutants was expressed in HEK293 cells and captured with the GFP binder (single chain lama antibody, produced in E.coli (Rothbauer et al., 2008)). This was precipitated with protein A beads. The precipitated proteins were separated by PAGE and Western-blotted. Blots were stained with anti-GFP antibodies to show successful pulldown and with U2AF-65 antibody for co-precipitated U2AF-65 (Figure 23).

The results indicate that N-terminal deletions of as little as seven amino acids are not tolerable for the interaction of U2AF-65 with Jmjd6. Moreover, the C-terminal region of Jmjd6 between amino acids 363 and 380 also appears indispensable for the interaction. This could indicate two binding sites for U2AF-65 on Jmjd6 located at opposite ends of the molecule. Alternatively, it is possible that oligomer formation of Jmjd6, which depends on the N-terminus (Figure 22), is necessary for its binding to U2AF-65. In any case, these data suggest sophisticated structural requirements for the Jmjd6-U2AF-65 complex to form. This notion is strengthened by the fact that active site mutants (ASM1 and 2), which are predicted to have lost the ability to complex Fe(II), also fail to bind to U2AF-65 (see Figure 23, lowest panels).

### Example 13: C-terminal deleted Jmjd6 localises in the nucleolus

In Figure 22E it was shown that deletion of the last 65 amino acids, which include the poly S-region of Jmjd6, resulted in a dramatic change of its localisation in the nucleus. In contrast to full length Jmjd6 the deletion mutant is almost exclusively nucleolar (Figure 24).

The dotted appearance of the signals in the nucleolus suggested a specific sub-nucleolar distribution. Three subregions can be distinguished in the nucleolus. These include the fibrillar centre (FC), where transcription of rRNAs takes place, the dense fibrillar component (DFC), that represent sites of pre-rRNA processing and modification, and the granular component (GC) where ribosomal subunits are assembled. In order to identify the precise nucleolar localisation of the Jmjd6 mutant co-staining experiments were performed with specific nucleolar antibodies, anti-UBF antibody for the FC (Dundr et al., 2002; Russell and Zomerdijk, 2005), anti-fibrillarin antibody for the DFC and anti-pescadillo for the GC (Lerch-Gaggl et al., 2002). As is shown in Figure 25, C-terminally deleted Jmjd6-GFP co-localises with UBF in the fibrillar centre of the nucleolus. This poses the question if Jmjd6 has a function in association with Pol1 transcription of rRNA genes. Moreover, these results indicate that nuclear/nucleolar distribution of PSR may be regulated via its C-terminus, possibly via the poly-serine region. A striking additional observation was that in cells where full length Jmjd6 is overexpressed UBF staining was lost (data not shown).

### Example 14: Jmjd6 overexpression changes SC-35 speckles and inhibits the formation of nascent transcripts

SC-35 is a marker protein for interchromatin clusters and perichromatin fibrils. These have a speckled appearance in immunofluorescence experiments with anti-SC-35 antibodies. They are highly dynamic nuclear compartments. Speckles turn into larger foci when Polymerase II-transcription is inhibited by α-amanitin. Phosphorylation is an important factor to modulate splicing activity, especially of SR proteins and it influences their distribution. Accumulation in foci is observed when phosphorylation is decreased by overexpression of mutated Clk1/STY (K190R) kinase. On the other hand, when SR proteins are hyperphosphorylated by phosphatase inhibition or overexpression of wt-kinase, they dissassemble. Here, the distribution of SC-35 in cells overexpressing Jmjd6 was analysed. The result is shown in Figure 26. In all cells where Jmjd6 was strongly overexpressed the SC-35 speckles were dramatically disassembled. In contrast, control cells that overexpressed GFP showed normal distribution of SC-35 in strong nuclear speckles (Figure 26). This result could hint at a connection of Jmjd6 activity and phosphorylation of splice factors. It was then investigated whether this dramatic change in SC-35 localisation in the nucleus after overexpression of Jmjd6 also influenced the appearance of native transcripts. A 5-FU labelling experiment was performed to label nascent transcript (Wansink et al., 1993);(Figure 27). Jmjd6 overexpressing cells showed global inhibition of 5-FU labelled native transcripts.

### Example 15: Jmjd6 changes splicing in a dual reporter splice assay

In addition to the splice assays performed by Natalia Gromak that detect alternative splicing of the tropomyosin gene (Webby et al, unpublished) a different splice assay was established, which includes expression of a constitutively spliced reporter construct. The double reporter system that has been described by Nasim et al in 2002 (Nasim et al., 2002) was used. It comprises a β-galactosidase gene, followed by an intron and a luciferase gene under control of an SV40 promoter. Upon splicing, a β-galactosidase-luciferase fusion protein is produced leading to quantifiable activities of both enzymes. In the absence of splicing, a stop codon within the intron prevents the expression of the luciferase gene and only β-galactosidase activity is observed. The advantage of this system is that only one plasmid is transfected. Therefore expression levels have no influence on the result concerning splicing of this reporter. Over-expression of Jmjd6 inhibited constitutive splicing of the reporter by ca. 50 % (Figure 28A). In order to exclude that the effect was not on splicing but on some later event in protein synthesis RT-PCR was also carried out to specifically amplify the spliced and non-spliced mRNAs (Figure 28B). It is shown that overexpression of Jmjd6 decreases the appearance of spliced mRNA (lane 1). Finally siRNA was used to knockdown Jmjd6. Two different si-RNAs (132 and 275) were used. Approximately 95 % of knockdown was achieved as judged by Western blotting (see Figure 30). In cells after Jmjd6 knockdown, splicing of the reporter was moderately increased (Figure 29). This assay therefore shows a consistent effect of Jmjd6 on constitutive splicing and should provide an important tool to elucidate the precise function of Jmjd6 during splicing.

### Example 16: Cell cycle arrest induced by Jmjd6 overexpression in HeLa cells

It was observed that HeLa cells after introduction of Jmjd6 slowed down their growth. Attempts to establish a stable cell line overexpressing Jmjd6 have failed so far. A few clones were obtained, but cells quickly lost Jmjd6 expression. Cell cycle progression was therefore analysed after transfection with plasmids from which Jmjd6 was overexpressed. FACS analysis of such cells is shown in Figure 31. GFP-Jmjd6 expressing cells had a diminished content of cells in S-phase in comparison with cells from the same transfection that did not express GFP-Jmjd6 (5 % in contrast to 26 %). This result was confirmed in an experiment where Jmjd6 expressing cells were analysed with the cell cycle marker PCNA (Leonhardt et al., 2000). S-phase cells show a very clear punctate PCNA distribution (see Figure 32, upper panel). In cells that overexpressed Jmjd6 this S-phase specific PCNA distribution could rarely be observed, again indicating failure of Jmjd6 cells to go into or proceed with S-phase (Figure 32, lower panel). These very preliminary experiments indicate that Jmjd6 causes cell cycle arrest in G1 or early S-phase.

### References for Examples 11 to 16

Dundr, M., Hoffmann-Rohrer, U., Hu, Q., Grummt, I., Rothblum, L. I., Phair, R. D. and Misteli, T. (2002). A kinetic framework for a mammalian RNA polymerase in vivo. Science 298, 1623-6.
Leonhardt, H., Rahn, H. P., Weinzierl, P., Sporbert, A., Cremer, T., Zink, D. and Cardoso, M. C. (2000). Dynamics of DNA replication factories in living cells. J Cell Biol 149, 271-80.
Lerch-Gaggl, A., Haque, J., Li, J., Ning, G., Traktman, P. and Duncan, S. A. (2002). Pescadillo is essential for nucleolar assembly, ribosome biogenesis, and mammalian cell proliferation. Journal of biological Chemistry 277,45347-45355.
Nasim, M. T., Chowdhury, H. M. and Eperon, I. C. (2002). A double reporter assay for detecting changes in the ratio of spliced and unspliced mRNA in mammalian cells. Nucleic Acids Research 30, e109/1-e109/6.
Rothbauer, U., Zolghadr, K., Muyldermans, S., Schepers, A., Cardoso, M. C. and Leonhardt, H. (2008). A versatile nanotrap for biochemical and functional studies with fluorescent fusion proteins. Mol Cell Proteomics 7, 282-9.
Russell, J. and Zomerdijk, J. C. (2005). RNA-polymerase-I-directed rDNA transcription, life and works. Trends Biochem Sci 30, 87-96.
Wansink, D. G., Schul, W., van der Kraan, I., van Steensel, B., van Driel, R. and de Jong, L. (1993). Fluorescent labeling of nascent RNA reveals transcription by RNA polymerase II in domains scattered throughout the nucleus. J Cell Biol 122, 283-93.
Zolghadr, K., Mortusewicz, O., Rothbauer, U., Kleinhans, R., Goehler, H., Wanker, E. E., Cardoso, M. C. and Leonhardt, H. (2008). A fluorescent two-hybrid assay for direct visualization of protein interactions in living cells. Mol Cell Proteomics 7, 2279-87.

### Informal Sequence Listing

SEQ ID NO: 1:

MNHKSKKRIR EAKRSARPEL KDSLDWTRHN YYESFSLSPA AVADNVERAD ALQLSVEEFV
ERYERPYKPV VLLNAQEGWS AQEKWTLERL KRKYRNQKFK CGEDNDGYSV KMKMKYYIEY
MESTRDDSPL YIFDSSYGEH PKRRKLLEDY KVPKFFTDDL FQYAGEKRRP PYRWFVMGPP
RSGTGIHIDP LGTSAWNALV QGHKRWCLFP TSTPRELIKV TRDEGGNQQD EAITWFNVIY
PRTQLPTWPP EFKPLEILQK PGETVFVPGG WWHVVLNLDT TIAITQNFAS STNFPVVWHK
TVRGRPKLSR KWYRILKQEH PELAVLADSV DLQESTGIAS DSSSDSSSSS SSSSSDSDSE
CESGSEGDGT VHRRKKRRTC SMVGNGDTTS QDDCVSKERS SSR

SEQ ID NO: 2

MNHKSKKRIR EAKRSARPEL KDSLDWTRHN YYESFSLSPA AVADNVERAD ALQLSVEEFV
ERYERPYKPV VLLNAQEGWS AQEKWTLERL KRKYRNQKFK CGEDNDGYSV KMKMKYYIEY
MESTRDDSPL YIFDSSYGEH PKRRKLLEDY KVPKFFTDDL FQYAGEKRRP PYRWFVMGPP
RSGTGIHIDP LGTSAWNALV QGHKRWCLFP TSTPRELIKV TRDEGGNQQD EAITWFNVIY
PRTQLPTWPP EFKPLEILQK PGETVFVPGG WWHVVLNLDT TIAITQNFAS STNFPVVWHK
TVRGRPKLSR KWYRILKQEH PELAVLADSV DLQESTGIAS DSS

SEQ ID NO: 3

DWTRHN YYESFSLSPA AVADNVERAD ALQLSVEEFV
ERYERPYKPV VLLNAQEGWS AQEKWTLERL KRKYRNQKFK CGEDNDGYSV KMKMKYYIEY
MESTRDDSPL YIFDSSYGEH PKRRKLLEDY KVPKFFTDDL FQYAGEKRRP PYRWFVMGPP
RSGTGIHIDP LGTSAWNALV QGHKRWCLFP TSTPRELIKV TRDEGGNQQD EAITWFNVIY
PRTQLPTWPP EFKPLEILQK PGETVFVPGG WWHVVLNLDT TIAITQNFAS STNFPVVWHK
TVRGRPKLSR KWYRILKQEH PELAVLADSV DLQESTGI

SEQ ID NO: 4

MSDFDEFERQ LNENKQERDK ENRHRKRSHS RSRSRDRKRR SRSRDRRNRD
QRSASRDRRR RSKPLTRGAK EEHGGLIRSP RHEKKKKVRK YWDVPPPGFE
HITPMQYKAM QAAGQIPATA LLPTMTPDGL AVTPTPVPVV GSQMTRQARR
LYVGNIPFGI TEEAMMDFFN AQMRLGGLTQ APGNPVLAVQ INQDKNFAFL
EFRSVDETTQ AMAFDGIIFQ GQSLKIRRPH DYQPLPGMSE NPSVYVPGVV
STVVPDSAHK LFIGGLPNYL NDDQVKELLT SFGPLKAFNL VKDSATGLSK
GYAFCEYVDI NVTDQAIAGL NGMQLGDKKL LVQRASVGAK NATLVSPPST
INQTPVTLQV PGLMSSQVQM GGHPTEVLCL MNMVLPEELL DDEEYEEIVE
DVRDECSKYG LVKSIEIPRP VDGVEVPGCG KIFVEFTSVF DCQKAMQGLT
GRKFANRVVV TKYCDPDSYH RRDFW

SEQ ID NO: 5

MSAQAQMRAM LDQLMGTSRD GDTTRQRIKF SDDRVCKSHL LNCCPHDVLS
GTRMDLGECL KVHDLALRAD YEIASKEQDF FFELDAMDHL QSFIADCDRR
TEVAKKRLAE TQEEISAEVA AKAERVHELN EEIGKLLAKV EQLGAEGNVE
ESQKVMDEVE KARAKKREAE EVYRNSMPAS SFQQQKLRVC EVCSAYLGLH
DNDRRLADHF GGKLHLGFIE IREKLEELKR VVAEKQEKRN QERLKRREER
EREEREKLRR SRSHSKNPKR SRSREHRRHR SRSMSRERKR RTRSKSREKR
HRHRSRSSSR SRSRSHQRSR HSSRDRSRER SKRRSSKERF RDQDLASCDR
DRSSRDRSPR DRDRKDKKRS YESANGRSED RRSSEEREAG EI

SEQ ID NO: 6

MISAAQLLDE LMGRDRNLAP DEKRSNVRWD HESVCKYYLC GFCPAELFTN
TRSDLGPCEK IHDENLRKQY EKSSRFMKVG YERDFLRYLQ SLLAEVERRI
RRGHARLALS QNQQSSGAAG PTGKNEEKIQ VLTDKIDVLL QQIEELGSEG
KVEEAQGMMK LVEQLKEERE LLRSTTSTIE SFAAQEKQME VCEVCGAFLI
VGDAQSRVDD HLMGKQHMGY AKIKATVEEL KEKLRKRTEE PDRDERLKKE
KQEREEREKE REREREERER KRRREEEERE KERARDRERR KRSRSRSRHS
SRTSDRRCSR SRDHKRSRSR ERRRSRSRDR RRSRSHDRSE RKHRSRSRDR
RRSKSRDRKS YKHRSKSRDR EQDRKSKEKE KRGSDDKKSS VKSGSREKQS
EDTNTESKES DTKNEVNGTS EDIKSEGDTQ S

## Claims

1. A method for assaying Jmjd6 activity, the method comprising contacting a splicing regulatory protein, or a fragment or variant thereof comprising an RS domain and a lysine residue, with a Jmjd6 polypeptide and determining whether the splicing regulatory protein or fragment thereof is hydroxylated.

2. The method of claim 1, wherein the splicing regulatory protein or fragment thereof and Jmjd6 polypeptide are contacted in the presence of Fe(II) and 2-oxoglutarate and optionally in the presence of a reducing agent.

3. The method of claim 1 or 2, wherein the splicing regulatory protein is the splicing factor U2AF 65 kDa subunit (U2AF65), Luc7-like2 or cisplatin resistance-associated overexpressed protein (CROP), or a fragment of any thereof comprising a lysine residue.

4. The method of any one of the preceding claims, wherein the RS domain comprises the sequence SRSR3CRRR wherein X is Asp or Glu.

5. The method of any one of the preceding claims, wherein the Jmjd6 polypeptide comprises:
(a) the amino acid sequence of SEQ ID NO: I,;
(b) a variant thereof having at least 60% identity to SEQ ID NO: 1 having lysyl hydroxylase activity; or
(c) a fragment of (a) or (b) of at least 200 amino acids in length which retains lysyl hydroxylase activity.

6. The method of any one of the preceding claims, wherein the assay is carried out in the presence of a test agent to determine whether the test agent is a modulator of Jmjd6 activity.

7. A method for identifying a modulator of RNA splicing, the method comprising contacting a cell which expresses Jmjd6 with a test agent and determining whether the test agent modulates Jmjd6 regulation of RNA splicing.

8. A method according to claim 7, wherein the cell comprises a RNA splicing reporter construct and the method comprises determining whether Jmjd6-mediated regulation of RNA splicing of the reporter construct is modulated by the test agent.

9. The method of any one of claims 6 to 8, wherein the test agent is a reported inhibitor of a 2-OG oxygenase other than Jmjd6.

10. The method of claim 9, wherein the inhibitor is an N-oxalyl amino acid, a glycine or alanine derivative, a 2-oxoacid analogue, a flavonoid or flavonoid derivative such as genistein.

11. The method of claim 6, wherein the method further comprises determining whether the test agent modulates activity of a 2-oxoglutarate dependent oxygenase other than Jmjd6, thereby determining whether the test agent selectively modulates Jmjd6 activity or selectively modulates activity of the 2-oxoglutarate dependent oxygenase other than Jmjd6.

## Patentansprüche

1. Verfahren zum Analysieren von Jmjd6-Aktivitä, wobei das Verfahren das Kontaktieren eines Spleissen regulierenden Proteins oder eines Fragments oder einer Variante desselben, das/die eine RS-Domäne und einen Lysinrest umfasst, mit einem Jmjd6-Polypeptid sowie das Ermitteln, ob das Spleissen regulierende Protein oder Fragment desselben hydroxyliert ist, umfasst.

2. Verfahren nach Anspruch 1, wobei das Spleissen regulierende Protein oder Fragment desselben und Jmjd6-Polypeptid bei Vorhandensein von Fe(II) und 2-Oxoglutarat und optional bei Vorhandensein eines Reduktionsmittels kontaktiert werden.

3. Verfahren nach Anspruch 1 oder 2, wobei das Spleissen regulierende Protein der Spleissfaktor U2AF 65 kDa Untereinheit (U2AF65), Luc7-like2 oder cisplatinresistenz-assoziiertes überexprimiertes Protein (CROP) oder ein Fragment eines derselben ist, das einen Lysinrest umfasst.

4. Verfahren nach einem der vorhergehenden Ansprüche, wobei die RS-Domäne die Sequenz SRSRXRRR umfasst, wobei X Asp oder Glu ist.

5. Verfahren nach einem der vorhergehenden Ansprüche, wobei das Jmjd6-Polypeptid umfasst:
(a) die Aminosäuresequenz von SEQ ID NO: 1;
(b) eine Variante davon mit mindestens 60% Identität zu SEQ ID NO: 1 mit Lysylhydroxylase-Aktivität; oder
(c) ein Fragment von (a) oder (b) mit einer Länge von mindestens 200 Aminosäuren, das Lysylhydroxylase-Aktivität beibehält.

6. Verfahren nach einem der vorhergehenden Ansprüche, wobei die Analyse bei Vorhandensein eines Testagens ausgeführt wird, um zu ermitteln, ob das Testagens ein Modulator von Jmjd6-Aktivität ist.

7. Verfahren zum Identifizieren eines Modulators von RNA-Spleissen, wobei das Verfahren das Kontaktieren einer Zelle, die Jmjd6 exprimiert, mit einem Testagens und das Ermitteln, ob das Testagens Jmjd6-Regulierung von RNA-Spleissen moduliert, umfasst.

8. Verfahren nach Anspruch 7, wobei die Zelle ein RNA spleissendes Reporterkonstrukt umfasst und das Verfahren das Ermitteln umfasst, ob eine Jmjd6-vermittelte Regulierung von RNA-Spleissen des Reporterkonstrukts durch das Testagens moduliert wird.

9. Verfahren nach einem der Ansprüche 6 bis 8, wobei das Testagens ein gemeldeter Inhibitor einer 2-OG-Oxygenase mit Ausnahme von Jmjd6 ist.

10. Verfahren nach Anspruch 9, wobei der Inhibitor eine N-oxalylaminosäure, ein Glycin- oder Alaninderivat, ein 2-Oxosäureanalog, ein Flavonoid oder Flavonoidderivat wie etwa Genistein ist.

11. Verfahren nach Anspruch 6, wobei das Verfahren weiterhin das Ermitteln umfasst, ob das Testagens eine Aktivität einer 2-Oxoglutarat-abhängigen Oxygenase mit Ausnahme von Jmjd6 moduliert, wodurch ermittelt wird, ob das Testagens eine Jmjd6-Aktivität selektiv moduliert oder eine Aktivität der 2-Oxoglutarat-abhängigen Oxygenase mit Ausnahme von Jmjd6 selektiv moduliert.

## Revendications

1. Méthode pour tester l'activité de Jmjd6, la méthode comprenant la mise en contact d'une protéine régulatrice de l'épissage, ou d'un fragment ou d'un variant de celle-ci comprenant un domaine RS et un résidu lysine, avec un polypeptide Jmdj6 et la détermination du fait que la protéine régulatrice de l'épissage ou un fragment de celle-ci est hydroxylée.

2. Méthode de la revendication 1, dans laquelle la protéine régulatrice de l'épissage ou un fragment de celle-ci et le polypeptide Jmjd6 sont mis en contact en présence de Fe(II) et de 2-oxoglutarate et éventuellement en présence d'un agent réducteur.

3. Méthode de la revendication 1 ou 2, dans laquelle la protéine régulatrice de l'épissage est la sous-unité de 65 kDa du facteur d'épissage U2AF (U2AF65), Luc7-de type 2 ou la protéine surexprimée associée à la résistance au cisplatine (CROP), ou un fragment d'un quelconque de ceux-ci comprenant un résidu lysine.

4. Méthode de l'une quelconque des revendications précédentes, dans laquelle le domaine RS comprend la séquence SRSRXRRR dans laquelle X est Asp ou Glu.

5. Méthode de l'une quelconque des revendications précédentes, dans laquelle le polypeptide Jmjd6 comprend .
(a) la séquence d'acides aminés de SEQ ID NO :1 ;
(b) un variant de celle-ci ayant au moins 60 % d'identité avec SEQ ID NO : 1 ayant une activité lysyl hydroxylase ; ou
(c) un fragment de (a) ou de (b) d'au moins 200 acides aminés de long qui conserve l'activité lysyl hydroxylase.

6. Méthode de l'une quelconque des revendications précédentes, dans laquelle l'essai est réalisé en présence d'un agent de test pour déterminer si l'agent de test est un modulateur de l'activité de Jmjd6.

7. Méthode d'identification d'un modulateur de l'épissage de l'ARN, la méthode comprenant la mise en contact d'une cellule qui exprime Jmjd6 avec un agent de test et la détermination du fait que l'agent de test module la régulation de l'épissage de l'ARN par Jmjd6.

8. Méthode selon la revendication 7, dans laquelle la cellule comprend une construction rapporteur de l'épissage de l'ARN et la méthode comprend la détermination du fait que la régulation de l'épissage de l'ARN de la construction rapporteur sous la médiation de Jmjd6 est modulée par l'agent de test.

9. Méthode de l'une quelconque des revendications 6 à 8, dans laquelle l'agent de test est un inhibiteur connu d'une 2-OG oxygénase autre que Jmjd6.

10. Méthode de la revendication 9, dans laquelle l'inhibiteur est un acide aminé N-oxalyl, un dérivé de glycine ou d'alanine, un analogue de 2-oxoacide, un flavonoïde ou un dérivé de flavonoïde, tel que la génistéine.

11. Méthode de la revendication 6, dans laquelle la méthode comprend en outre la détermination du fait que l'agent de test module l'activité d'une oxygénase 2-oxoglutarate dépendante autre que Jmjd6, déterminant ainsi si l'agent de test module sélectivement l'activité de Jmjd6 ou module sélectivement l'activité de l'oxygénase 2-oxoglutarate dépendante autre que Jmjd6.
